(19) Europäisches Patentamt
European Patent Office
Office européen des brevets

(11) **EP 3 970 149 B1**

(12) **EUROPEAN PATENT SPECIFICATION**

(45) Date of publication and mention
of the grant of the patent:
**07.01.2026 Bulletin 2026/02**

(21) Application number: **20723173.9**

(22) Date of filing: **08.05.2020**

(51) International Patent Classification (IPC):
**G16B 5/00** (2019.01)   **C12Q 1/6886** (2018.01)
**G16H 50/20** (2018.01)   **G16H 50/50** (2018.01)

(52) Cooperative Patent Classification (CPC):
**G16B 5/00; C12Q 1/6886; G16H 50/20;**
**G16H 50/50;** C12Q 2600/158

(86) International application number:
**PCT/EP2020/062925**

(87) International publication number:
**WO 2020/229361 (19.11.2020 Gazette 2020/47)**

(54) **ASSESSMENT OF MULTIPLE SIGNALING PATHWAY ACTIVITY SCORE IN AIRWAY EPITHELIAL CELLS TO PREDICT AIRWAY EPITHELIAL ABNORMALITY AND AIRWAY CANCER RISK**

BEURTEILUNG DES AKTIVITÄTS-SCORES MEHRERER SIGNALISISERUNGSPFADE IN ATEMWEGSEPITHELZELLEN ZUR VORHERSAGE DER ATEMWEGSEPITHELANOMALITÄT UND DES ATEMWEGSKREBSRISIKOS

ÉVALUATION DU SCORE DE L'ACTIVITÉ DES VOIES DE SIGNALISATION MULTIPLES DANS DES CELLULES ÉPITHÉLIALES DES VOIES RESPIRATOIRES POUR PRÉDIRE UNE ANOMALIE ÉPITHÉLIALE DES VOIES RESPIRATOIRES ET LE RISQUE DE CANCER DES VOIES RESPIRATOIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.05.2019 EP 19174032**

(43) Date of publication of application:
**23.03.2022 Bulletin 2022/12**

(73) Proprietor: **Koninklijke Philips N.V.**
**5656 AG Eindhoven (NL)**

(72) Inventors:
• **VAN DE STOLPE, Anja**
**5656 AE Eindhoven (NL)**
• **JACOBS, Igor**
**5656 AE Eindhoven (NL)**
• **KUIJPERS, Steven, Paulus, Lambertus**
**5656 AE Eindhoven (NL)**

(74) Representative: **Philips Intellectual Property & Standards**
**High Tech Campus 52**
**5656 AG Eindhoven (NL)**

(56) References cited:
**WO-A1-2016/062891   WO-A1-2019/068623**

• **ANJA VAN DE STOLPE: "Quantitative Measurement of Functional Activity of the PI3K Signaling Pathway in Cancer", CANCERS, vol. 11, no. 3, 1 March 2019 (2019-03-01), pages 293, XP055642149, DOI: 10.3390/cancers11030293**
• **XUN YUAN ET AL: "Notch signaling and EMT in non-small cell lung cancer: biological significance and therapeutic application", JOURNAL OF HEMATOLOGY & ONCOLOGY, BIOMED CENTRAL LTD, LONDON UK, vol. 7, no. 1, 5 December 2014 (2014-12-05), pages 87, XP021206357, ISSN: 1756-8722, DOI: 10.1186/ S13045-014-0087-Z**

- ANN E. TILLEY ET AL: "Down-regulation of the Notch Pathway in Human Airway Epithelium in Association with Smoking and Chronic Obstructive Pulmonary Disease", AMERICAN JOURNAL OF RESPIRATORY AND CRITICAL CARE MEDICINE, vol. 179, no. 6, 15 March 2009 (2009-03-15), pages 457 - 466, XP055122525, ISSN: 1073-449X, DOI: 10.1164/rccm.200705-795OC
- JAE-WOONG HWANG ET AL: "FOXO3 Deficiency Leads to Increased Susceptibility to Cigarette Smoke-Induced Inflammation, Airspace Enlargement, and Chronic Obstructive Pulmonary Disease", THE JOURNAL OF IMMUNOLOGY, vol. 187, no. 2, 20 June 2011 (2011-06-20), US, pages 987 - 998, XP055706827, ISSN: 0022-1767, DOI: 10.4049/jimmunol.1001861
- D. SAMANTA ET AL: "Smoking Attenuates Transforming Growth Factor- -Mediated Tumor Suppression Function through Downregulation of Smad3 in Lung Cancer", CANCER PREVENTION RESEARCH, vol. 5, no. 3, 9 January 2012 (2012-01-09), United States, pages 453 - 463, XP055706823, ISSN: 1940-6207, DOI: 10.1158/1940-6207.CAPR-11-0313
- "AFFYMETRIX CATALOG; PRODUKT: HUMAN GENOME U133A ARRAY; ARRAY FINDER", AFFYMETRIX PRODUCT CATALOG, XX, XX, 1 July 2002 (2002-07-01), pages 1, XP002267612

**Description**

FIELD OF THE INVENTION

The subject-matter described herein mainly relates to bioinformatics, genomic processing arts, proteomic processing arts, and related arts. More particularly, the present invention relates to a computer-implemented method for determining whether a subject has abnormal airway epithelium, and to a computer-implemented method for determining a risk score that indicates a risk that a subject having abnormal airway epithelium will develop an airway cancer. The present invention further relates to an apparatus, a non-transitory storage medium and a computer program, for determining an airway abnormality factor indicating whether a subject has abnormal airway epithelium or a risk score that indicates a risk that a subject having abnormal airway epithelium will develop an airway cancer. The present invention further relates to a kit for determining an airway abnormality factor indicating whether a subject has abnormal airway epithelium or for determining a risk score that indicates a risk that a subject having abnormal airway epithelium will develop an airway cancer as well as a kit for use in a corresponding method of diagnosing or prognosticating. The airway abnormality factor and the risk score are determined based on a combination of signaling pathway activities.

BACKGROUND OF THE INVENTION

Lung cancer is a deadly disease with a poor prognosis and the leading cause of cancer mortality worldwide. The majority of patients is diagnosed at an advanced stage and 5-year survival rates are only 18.6%. Therefore, early detection and timely diagnosis, staging and treatment are essential in lung cancer care. Screening programs are directed at early detection of lung cancer, sometimes in high risk groups, aiming at improving clinical outcome by installing therapy in an early stage. Low-dose CT (LDCT) screening of high risk populations is being implemented, in which screening eligibility is primarily based on smoking history. However, one of the drawbacks of LDCT screening is the relatively high false-positive rates (NLST Research Team. N Engl J Med; 368:1980-1991 (2013)), as well as exposition to a cumulative radiation dose in case of repeated screening procedures. Positive CT findings require additional follow-up, using potentially unnecessary biopsy procedures to obtain pathologic confirmation -with associated health risks for the patient at hand. Depending on the location of the tumor it may be difficult to obtain a tumor sample for diagnostic/subtyping purposes. Hence, there is a need for tests for early detection of lung cancer with higher sensitivity/specificity, and without radiation exposure. Also minimally invasive tests that identify a high risk population for subsequent next step imaging analysis are needed, to reduce the number of unnecessary CT scans and consequent false positives. Also, if a nodule is found on for example a CT scan, a decision needs to be taken as to it is necessary to take a biopsy to enable a pathology diagnosis. This is an invasive and risky procedure. Therefore, there is a need for methods that help to take this decision by providing complementary evidence as to the probable cause of the identified nodule, i.e., benign or malignant.

The most common type of lung cancer is non-small cell lung carcinoma (NSCLC), of which the most common subtypes are adenocarcinoma (40%) and squamous cell carcinoma (30%). Cigarette smoking accounts for about 85% of all lung cancers. Roughly between 5 and 15% of smokers will develop lung cancer. Patient with COPD, which is associated with (heavy) smoking, are at increased risk to develop lung cancer (Oncotarget. 2017 Sep 29; 8(44): 78044-78056; Lung Cancer. 2015 Nov; 90(2): 121-127; Respiration. 2011; 81(4):265-84).

Tobacco exposure is an important factor in the induction of pre-malignant changes. The vast majority of lung cancers is associated with smoking, although 15% occur in never smokers. Especially heavy smoking is associated with increased risk at lung cancer, specifically the squamous cell and small cell type of lung cancer but also the other histopathological cancer types.

In never-smokers, adenocarcinoma is the predominant subtype and squamous cell carcinoma in never-smokers is rare. Adenocarcinoma often has a peripheral or endobronchial origin, whereas squamous cell carcinoma usually arises in the trachea or proximal airways.

Tobacco exposure induces stress to airway epithelial cells and may cause smoking-induced injury that requires epithelial regeneration and repair. Smoking interferes with the signaling pathways that are involved in these processes, which may promote tumorigenicity.

Pre-malignant lesions in general, for example colon adenomas, but also in airway epithelium, are characterized by increased proliferation, reflected in abnormal activity of certain signal transduction pathways, such as the PI3K patwhay (Clin Cancer Res. 2018 Jul 1;24(13):2984-2992; Sci Transl Med. 2010 Apr 7;2(26):26ra25). Abnormal proliferation can be enabled by activity of one or more growth factor pathways, like the PI3K pathway or the MAPK-AP1 or JAK-STAT pathways, for example associated with loss of pathway activities which in normal cells restrain/control cell proliferation, like the TGF-$\beta$ or Notch pathway. During evolution to cancer other oncogenic signal transduction pathways can be recruited or more controlling pathways lost, due to an increasing number of genomic mutations and/or chromosome aberrations. Thus, the pre-malignant lesion may share some pathway activity characteristics, but not necessarily all with the final cancer. Maybe for this reason little is known with respect to roles of signal transduction pathways in (lung) cancer development and interference of smoking with pathway activity.

Obtaining an epithelial sample from the large or small airways for analysis: Analysis of a sample from the epithelial lining of the airways may provide information on premalignant changes, or provide additional diagnostic information in case a suspect nodule is seen on for example a CT scan.

Lung cancer develops in the larger airway (bronchi branching off the trachea) or small airway epithelium. Using techniques like airway brushing or broncho-alveolar lavage, epithelial cells can be obtained for molecular analysis from upper and lower airways in a relatively non-invasive manner. This provides a potential means to identify individuals, i.e., smokers, that have abnormal oncogenic signaling pathway activity in their airway epithelium, that are indicative of early proliferative changes in airway epithelium and may be at increased risk for developing lung cancer. COPD patients are generally chronic heavy smokers and constitute a high risk group for development of lung cancer.

Abnormal signal transduction pathway activities, when identified, can in principle be regulated by targeted drugs that target a specific signaling pathway, e.g. blocked by PI3K pathway inhibitors (W Verhaegh et al., Cancer research, 2014; 74(11):2936-45; A van de Stolpe et al., Scientific Reports, 2019, 9(1603); van Ooijen, Am J Pathol, 2018, 188(9):1956-1972). This opens the option that pre-malignant airway epithelial lesions are clinically actionable and that the abnormality can be reversed or eliminated upon local treatment of the airway epithelium.

WO 2016/062891 A1 relates to methods determining TGFbeta activity in a sample from a patient, and prognosis based on the determined pathway activity. Tilley et al. (m J Respir Crit Care Med. 2009 Mar 15;179(6):457-66.) describe thtat the Notch pathway is down-regulated in human airway epithelium in association with smoking and chronic obstructive pulmonary disease. Hwang et al. (J Immunol. 2011 Jul 15;187(2):987-98.) describe that FOXO3 deficiency leads to increased susceptibility to cigarette smoke-induced inflammation, airspace enlargement, and chronic obstructive pulmonary disease. Samanta et al. (Cancer Prev Res (Phila). 2012 Mar;5(3):453-63) disclose that smoking attenuates Transforming growth factor-$\beta$-mediated tumor suppression function through down-regulation of Smad3 in lung cancer.

Hence, there is a high need for a method to detect early changes in airway epithelium that are indicative for abnormal epithelium, and characterizes the abnormality in terms of aberrant signaling pathway activity. The method is expected to be of use for identification of individuals at high risk for lung cancer, and to provide addition evidence for a malignant versus benign character of an identified lung nodule, and to indicate which therapy may be useful to treat the identified lesion or associated lung cancer.

SUMMARY OF THE INVENTION

In accordance with a first aspect of the present invention, the above problem is solved by a computer-implemented method for determining whether a subject has abnormal airway epithelium, performed by a digital processing device, wherein the determining comprises determining an airway abnormality factor indicating whether the subject has abnormal airway epithelium based on a combination of activities of cellular signaling pathways in an epithelial cell sample derived from an airway of the subject, wherein the cellular signaling pathways comprises two or more cellular signaling pathways selected from the group consisting of a TGF-$\beta$ pathway, a PI3K-FOXO pathway, and a Notch pathway, wherein the determining of the signaling pathway abnormality factor is further based on a reference activity of the respective cellular signaling pathway, wherein the reference activity reflects activity of the respective cellular signaling pathway found in airway epithelium of healthy subjects, wherein the determining of the airway abnormality factor comprises: determining a signaling pathway abnormality factor for each of the respective cellular signaling pathways based on the activity of the respective cellular signaling pathway in the epithelial cell sample and determining the airway abnormality factor based on a combination of the determined cellular signaling pathway abnormality factors, wherein a quantitative measurement of signal transduction pathway activity in epithelial cells obtained from upper or lower airways is based on inferring activity of a signal transduction pathway from measurements of mRNA levels of three or more validated direct target genes of the transcription factor associated with the respective signaling pathway. Additional cellular signaling pathways may be optionally included in the method of the present invention. In one embodiment, the method further comprises providing the airway abnormality factor for the purpose of any one of the various uses disclosed herein, such as determining a risk score that indicates a risk that a subject having abnormal airway epithelium will develop an airway cancer, defining an interval, after which the method shall be repeated, recommending supplementary diagnosis to be performed, recommending treatment or the like.

According to a second aspect of the present invention, the problem is solved by a computer-implemented method for determining a risk score that indicates a risk that a subject having abnormal airway epithelium will develop an airway cancer performed by a digital processing device, wherein the determining comprises determining the risk score based on a combination of the activities of cellular signaling pathways in an epithelial cell sample derived from an airway of the subject, wherein the cellular signaling pathway analysis comprises two or more cellular signaling pathways selected from the group consisting of a TGF-$\beta$ pathway, a PI3K-FOXO pathway, and a Notch pathway, wherein the determining of the risk score is further based on a combination of reference activities of the cellular signaling pathways, and wherein the risk score is defined such that the indicated risk increases with a decreasing activity of the TGF-$\beta$ pathway and one or more of an increasing activity of the PI3K pathway, and/or a decreasing activity of the Notch pathway with respect to the reference activities of the cellular signaling pathways, wherein the determining of the risk score comprises: determining an airway abnormality factor based on the combination of the activities of the cellular signaling pathways in the epithelial cell sample and translating the airway abnormality factor into the risk score, wherein the determining of the airway abnormality factor comprises: determining a signaling pathway abnormality factor for each of the respective cellular signaling pathways based on the activity of the respective cellular signaling pathway in the epithelial cell sample and determining the airway abnormality factor based on a combination of the determined cellular signaling pathway abnormality factors, wherein a quantitative measurement of signal transduction pathway activity in epithelial cells obtained from upper or lower airways is based on inferring activity of a signal transduction pathway from measurements of mRNA levels of three or more validated direct target genes of the transcription factor associated with the respective signaling pathway. In one embodiment, the method further comprises providing the risk score for the purpose of any one of the various uses disclosed herein, such as defining an interval, after which the method shall be repeated, recommending a supplementary diagnostic method to be performed, recommending treatment or the like.

The present invention is based on the inventor's innovation that analysis of signal transduction pathway activities can be used to characterize even pre-malignant chances of airway epithelium that play a role in the development towards a malignant airway cancer. The inventors found that the presence or absence of malignant and pre-malignant chances can be assessed by measuring activities of certain signaling pathways and evaluate the measured activities in combination. The inventors for the first time provide insight how the relevant pathway activities are related to each other in determining whether airway epithelium is abnormal and/or whether an abnormal airway epithelium characterizes a subject that is at risk of developing an airway cancer.

The present invention has been accomplished by intensively studying the activities of signaling pathways (in particular TGF-β, PI3K-FOXO, Notch pathways) in airway epithelial cells of subjects belonging to different groups including healthy non-smokers, light smokers and heavy smokers. Subsequently, a computational model has been developed for interpretation of the measured pathway activities in order to determine abnormal deviations from normal pathway activities, which can be indicated by a signaling pathway abnormality factor, and provide an airway abnormality factor and/or risk score of a subject having an unknown characteristic of the airway epithelium. In this model, the combined activities of the signaling pathways are used as an indicator (biomarker) that characterizes the likelihood that a subject has abnormal airway epithelium and/or is at risk of developing an airway cancer.

As an advantage, the present invention facilitates identification of a subject that is at risk of developing cancer at an early stage of the disease and therefore enables better treatment options than existing methods. It is expected that use of this method will reduce the number of unnecessary CT scans, thereby reducing the numebr of unnecessary invasive diagnostic procedures and exposure to harmful radiation. Lung cancer development is a multistep process, in which normal lung epithelial cells accumulate genetic abnormalities and transform into malignant phenotypes. The methods of the present invention have a huge potential to aid in detection of early (pre-malignant) changes that will finally tum into malignant chances at a later stage and may in this context be applicable in various clinical scenarios. Before an airway cancer is detectable, the methods of the present invention may be used for risk assessment and identification of populations that may benefit from screening or primary chemoprevention, or local treatment to block progression or reverse the lesion to normal. Furthermore, as mentioned the methods of the present invention may be used to detect lung cancer in a pre-malignant or early stage, before progression to invasive lung cancer. The methods of the present invention may aid in selection of individuals for close CT monitoring and secondary chemoprevention. In addition, the methods of the present invention may be of value to differentiate between benign and malignant nodules found on CT images and aid in prognostication. Finally, some biopsy procedures may have limited sensitivity and therefore not always result in establishment of a diagnosis. For example, bronchoscopic examinations can frequently be non-diagnostic, requiring additional invasive testing. Combination of such known diagnostic tests with the methods of the present invention is expected to significantly improve their diagnostic performance. In case of finding a non-siagnostic nodule on for example a CT scan, the method will be of help in classifying the nodule as malignant or benign.

The term "subject", as used herein, refers to any living being. In some embodiments, the subject is an animal, preferably a mammal. In certain embodiments, the subject is a human being, such as a medical subject. Although the applicability of the methods of the present invention is not limited to a particular group of subjects, it will be apparent that a subject belonging to a high risk group such as smoker or COPD profits most of the invention. It is therefore preferred that the subject to be diagnosed is a smoker, in particular a tobacco smoker, or a subject that is or has been regularly exposed to smoke, for example a subject that lives in the same household as a smoker. Other risk factors that preferably define the subject to be diagnosed include exposure to radon, asbestos, arsenic, diesel exhaust, silica and chromium. The methods of the invention may be advantageously applied repeatedly, in particular in regular intervals so that pre-malignant changes of airway epithelium can be detected as early as possible.

The epithelial cell sample to be used in accordance with the present invention can be an extracted sample, that is, a sample that has been extracted from the subject. Examples of the sample include, but are not limited to epithelial cells, tissue and/or body fluid containing epithelial cells from the subject's airway. The epithelial cell sample may also be a sample comprising progenitor and/or stem cells of epithelial cells such as basal cells (BC) that constitute the stem/progenitor cells needed for regeneration of damaged epithelium. The respective sample can for example be obtained from the subject's upper or lower airway by broncho-alveolar lavage, brushing, biopsy or the like. The term "sample", as used herein, also encompasses the case where e.g. cells, tissue and/or body fluid have been taken from the subject and, e.g., have been put on a microscope slide or fixative, and where for performing the claimed method a portion of this sample is extracted, e.g., by means of Laser Capture Microdissection (LCM), or by punching, or by scraping off the cells of interest from the slide, or by fluorescence-activated cell sorting techniques. In addition, the term "sample", as used herein, also encompasses the case where e.g. cells, tissue and/or body fluid have been taken from the subject and have been put on a microscope slide, and the claimed method is performed on the slide.

The term "upper airway" as used herein includes nasal cavity, pharynx (including nasopharynx, oropharynx and laryngopharynx) and larynx. The term "lower airway" as used herein includes trachea and lungs (including primary bronchi and bronchioles).

The terms "pathway", "signal transduction pathway", "signaling pathway" and "cellular signaling pathway" are used interchangeably herein.

An "activity of a signaling pathway" may refer to the activity of a signaling pathway associated transcription factor (TF) element in the sample, the TF element controlling transcription of target genes, in driving the target genes to expression, i.e., the speed by which the target genes are transcribed, e.g. in terms of high activity (i.e. high speed) or low activity (i.e. low speed), or other dimensions, such as levels, values or the like related to such activity (e.g. speed). Accordingly, for the purposes of the present invention, the term "activity", as used herein, is also meant to refer to an activity level that may be obtained as an intermediate result during "pathway analysis" as described herein.

The term "transcription factor element" (TF element), as used herein, preferably refers to an intermediate or precursor protein or protein complex of the active transcription factor, or an active transcription factor protein or protein complex which controls the specified target gene expression. For example, the protein complex may contain at least the intracellular domain of one of the respective signaling pathway proteins, with one or more co-factors, thereby controlling transcription of target genes. Preferably, the term refers to either a protein or protein complex transcriptional factor triggered by the cleavage of one of the respective signaling pathway proteins resulting in a intracellular domain.

The term "target gene", as used herein, means a gene whose transcription is directly or indirectly controlled by a respective transcription factor element. The "target gene" may be a "direct target gene" and/or an "indirect target gene" (as described herein).

The terms "abnormal" and "abnormality" as used herein denote a characteristic assigned to a subject, a cell, or a cell sample, that is regarded as rare, dysfunctional, malfunctional or malignant, and in particular characterize a "pre-malignant" change in a subject, cell, or cell sample. Diagnosis of an abnormal (pre-malignant) characteristic may indicate a subject to be at risk of evolution towards a malignant airway cancer. For the purposes of the present invention, a subject, a cell, or a cell sample, is in particular considered to be abnormal, if it has been determined, based on respective pathway activities as disclosed herein, to deviate from respective reference pathway activities. The reference pathway activities are in particular those pathway activities that can be found in a healthy (i.e. normal) subject, a cell from a healthy subject or a sample comprising cells from a healthy subject. If not already known, the reference pathway activities can be empirically determined by pathway analysis as disclosed herein using an epithelial cell sample of one or more healthy subjects. Preferably, samples from a plurality of healthy subjects are assessed to take account of natural pathway activity variation.

In accordance with this, the present invention describes that the determining of the airway abnormality factor and/or the risk score is further based on a respective combination of reference activities of signaling pathways. Similarly, the determining of the signaling pathway abnormality factor may be further based on a reference activity of the respective signaling pathway. A reference activity reflects activity of the respective signaling pathway found in airway epithelium of healthy subjects.

By comparing each of the reference pathway activities to each of the respective pathway activities in the subject to be diagnosed, signaling pathway abnormality factors for each of the respective pathways can be determined. The signaling pathway abnormality factor indicates whether the activity of the respective pathway deviates (abnormally) from the reference activity of the respective pathway. The signaling pathway abnormality factors may then be translated into an airway abnormality factor. The airway abnormality factor may also be computed directly from the combination of pathway activities. The airway abnormality factor can be considered as multi-pathway score, MPS, and denotes a likelihood that a subject has abnormal airway epithelium. Accordingly, the "airway abnormality factor", herein also referred to as "airway pathway abnormality score" (APAS), or specifically as "small airway pathway abnormality score" (SAPAS)" or "large airway pathway abnormality score" (LAPAS), refers to a dimension, e.g. a level or a value, relating the combination of pathway activities to a likelihood that the subject has abnormal (e.g. small or large) airway epithelium.

The term "airway cancer" as used herein refers to a malignant tumor of the airway and in this context in particular to cancer types for which the risk increases when you smoke. The method could be broadly applicable to these cancer types, especially when they originate from epithelial cells. The present invention focuses on cancers originating from epithelial cells of the airways. Non-limiting examples of such cancers include tracheal cancers, bronchial cancers, cancer of the upper airways (including nasal, oral, laryngeal, etc.), lung cancer, subtypes of lung cancer including non-small cell lung carcinoma (NSCLC) and small cell lung carcinoma (SCLC), histologic subtypes such as squamous lung cancer, adenosquamous carcinoma, large cell carcinoma, sarcomatoid carcinoma and lung adenocarcinoma. The risk score can be determined by comparing each of the reference pathway activities to each of the respective pathway activities in the epithelial cell sample of the subject to be diagnosed. The risk score can be considered as multi-pathway score, MPS, and denotes a likelihood that a subject will develop an airway cancer. Accordingly, the "risk score" refers to a dimension, e.g. a level or a value, relating the combination of pathway activities to a likelihood that the subject will develop an airway cancer.

The airway abnormality factor and/or risk factor is based on a "combination of activities of cellular signaling pathways". This means that the airway abnormality factor and/or risk factor is influenced by the activities of two or more cellular signaling pathways. The activities of the two or more cellular signaling pathways can be inferred and/or combined by a mathematical model as described herein. **In** a preferred embodiment, the airway abnormality factor and/or risk score is based on a combination of signaling pathway activities comprising activities of more than 2 cellular signaling pathways. Such combination of signaling pathway activities may include the activities of 3 or 4, or even more than 4 such as 5, 6, 7 or 8, or even more, different signaling pathways.

**In** general, many different formulas can be devised for determining an airway abnormality factor and/or risk score that is based on a combination of activities of two or more cellular signaling pathways in a subject, i.e.:

$$MPS = F(Pi) + X, \text{ with } i = 1...N,$$

wherein MPS denotes the airway abnormality factor and/or risk score (the term "MPS" is used herein as an abbreviation for "Multi-Pathway Score" in order to denote that the risk score is influenced by the activities of two or more cellular signaling pathways), Pi denotes the activity of cellular signaling pathway i, N denotes the total number of cellular signaling pathways used for calculating the airway abnormality factor and/or risk score, and X is a placeholder for possible further factors and/or parameters that may go into the equation. Such a formula may be more specifically a polynomial of a certain degree in the given variables, or a linear combination of the variables. The weighting coefficients and powers in such a polynomial may be set based on expert knowledge, but typically a training data set with known ground truth, e.g., survival data, is used to obtain estimates for the weighting coefficients and powers of the formula above. The activities may be combined using the formula above and will subsequently generate an MPS. Next, the weighting coefficients and powers of the scoring function may be optimized such that a high MPS correlates with a higher probability that the patient will have abnormal airway epithelium and/or will develop and airway cancer, and vice versa. Optimizing the scoring function's correlation with known data can be done using a multitude of analysis techniques, e.g., a Cox proportional hazards test (as preferably used herein), a log-rank test, a Kaplan-Meier estimator in conjunction with standard optimization techniques, such as gradient-descent or manual adaptation, and so on.

According to a preferred embodiment of the invention, the airway abnormality factor and the risk score, respectively, is determined based on evaluating a calibrated mathematical model relating the activities of the signaling pathways in the epithelial cell sample to the airway abnormality factor and the risk score, respectively. This model may be programmed to interpret the combination of pathway activities so as to determine the airway abnormality factor and/or the risk score of the subject to be diagnosed. In particular, the determination of the airway abnormality factor and/or the risk score comprises (i) receiving activity of the respective signaling pathways in the epithelial cell sample of the subject to be diagnosed, (ii) determining the airway abnormality factor and/or the risk score of said subject, the determining being based on evaluating a calibrated mathematical model relating the activity of the respective signaling pathways to the airway abnormality factor and/or the risk score.

The calibrated mathematical pathway model is preferably a centroid or a linear model, or a Bayesian network model based on conditional probabilities. For example, the calibrated mathematical pathway model may be a probabilistic model, preferably a Bayesian network model, based on conditional probabilities relating the airway abnormality factor and/or the risk score and the activities of the signaling pathways, or the calibrated mathematical pathway model may be based on one or more linear combination(s) of the activities of the signaling pathways.

In accordance with the mathematical model, the activities of the signaling pathways are interpreted to provide the airway abnormality factor, which may further be translated into the risk score, or are interpreted to provide directly the risk score. The airway abnormality factor and risk score predict or provide a probability that a subject has abnormal airway epithelium and/or that a subject is at risk of developing an airway cancer.

Accordingly, the determining of the risk score may comprise determining an airway abnormality factor based on the combination of the activities of the cellular signaling pathways in the epithelial cell sample and translating the airway abnormality factor into the risk score. The determining of the airway abnormality factor may comprise determining a signaling pathway abnormality factor for each of the respective cellular signaling pathways based on the activity of the respective cellular signaling pathway in the epithelial cell sample and determining the airway abnormality factor based on a combination of the determined signaling pathway abnormality factors

According to a preferred embodiment of the present invention, the activity of the respective signal pathway is determined or determinable by pathway analysis as described herein.

Pathway analysis enables quantitative measurement of signal transduction pathway activity in epithelial cells, based on inferring activity of a signal transduction pathway from measurements of mRNA levels of the well-validated direct target genes of the transcription factor associated with the respective signaling pathway (see for example W Verhaegh et al., 2014, *supra;* W Verhaegh, A van de Stolpe, Oncotarget, 2014, 5(14):5196).

Preferably the determining of the activities of the signaling pathways, the combination of multiple pathway activities and applications thereof is performed as described for example in the following documents: published international patent applications WO2013011479 (titled "ASSESSMENT OF CELLULAR SIGNALING PATHWAY ACTIVITY USING PROBABILISTIC MODELING OF TARGET GENE EXPRESSION"), WO2014102668 (titled "ASSESSMENT OF CELLULAR SIGNALING PATHWAY ACTIVITY USING LINEAR COMBINATION(S) OF TARGET GENE EXPRESSIONS"), WO2015101635 (titled "ASSESSMENT OF THE PI3K CELLULAR SIGNALING PATHWAY ACTIVITY USING MATHEMATICAL MODELLING OF TARGET GENE EXPRESSION"), WO2016062891 (titled "ASSESSMENT OF TGF-$\beta$ CELLULAR SIGNALING PATHWAY ACTIVITY USING MATHEMATICAL MODELLING OF TARGET GENE EXPRESSION"), WO2017029215 (titled "ASSESSMENT OF NFKB CELLULAR SIGNALING PATHWAY ACTIVITY USING MATHEMATICAL MODELLING OF TARGET GENE EXPRESSION"), WO2014174003 (titled "MEDICAL PROGNOSIS AND PREDICTION OF TREATMENT RESPONSE USING MULTIPLE CELLULAR SIGNALLING PATHWAY ACTIVITIES"), WO2016062892 (titled "MEDICAL PROGNOSIS AND PREDICTION OF TREATMENT RESPONSE USING MULTIPLE CELLULAR SIGNALING PATHWAY ACTIVITIES"), WO2016062893 (titled "MEDICAL PROGNOSIS AND PREDICTION OF TREATMENT RESPONSE USING MULTIPLE CELLULAR SIGNALING PATHWAY ACTIVITIES"), WO2018096076 (titled "Method to distinguish tumor suppressive FOXO activity from oxidative stress"), and in the patent applications EP16200697.7 (filed on Nov 25, 2016; titled "Method to distinguish tumor suppressive FOXO activity from oxidative stress"), EP17194288.1 (filed on Oct 2, 2017; titled "Assessment of Notch cellular signaling pathway activity using mathematical modelling of target gene expression"), EP17194291.5 (filed on Oct 2, 2017; titled "Assessment of JAK-STAT1/2 cellular signaling pathway activity using mathematical modelling of target gene expression"), EP17194293.1 (filed on Oct 2, 2017; titled "Assessment of JAK-STAT3 cellular signaling pathway activity using mathematical modelling of target gene expression") and EP17209053.2 (filed on Dec 20, 2017, titled "Assessment of MAPK-AP1 cellular signaling pathway activity using mathematical modelling of target gene expression"), PCT/EP2018/076232 (filed on Sep 27, 2018, titled "Assessment of JAK-STAT3 cellular signaling pathway activity using mathematical modelling of target gene expression"), PCT/EP2018/076334 (filed on Sep 27, 2018, titled "Assessment of JAK-STAT1/2 cellular signaling pathway activity using mathematical modelling of target gene expression"), PCT/EP2018/076488 (filed on Sep 28, 2018, titled "Assessment of Notch cellular signaling pathway activity using mathematical modelling of target gene expression"), PCT/EP2018/076513 (filed on Sep 28, 2018, titled "Assessment of MAPK-AP-1 cellular signaling pathway activity using mathematical modelling of target gene expression"), and PCT/EP2018/076614 (filed on Oct 1, 2018, titled "Determining functional status of immune cells types and immune response").

The models have been biologically validated for ER, AR, PI3K-FOXO, HH, Notch, TGF-$\beta$, Wnt, NFkB, JAK-STAT1/2, JAK-STAT3 and MAPK-AP1 pathways on several cell types. It is noted that the mathematical models employed in the patent applications that are not yet published as well as the calibration and use of these models in these applications generally correspond to the models, calibration and use disclosed in the already published patent applications.

Unique sets of cellular signaling pathway target genes whose expression levels are preferably analyzed have been identified. For use in the mathematical models, three or more, for example, three, four, five, six, seven, eight, nine, ten, eleven, twelve or more, target genes from each assessed cellular signaling pathway can be analyzed to determine pathway activities.

Common to the pathway analysis methods for determining the activities of the different signaling pathways as disclosed herein is a concept, which is preferably applied herein for the purposes of the present invention, wherein the activity of a signaling pathway in a cell such as an epithelial cell present in a sample is determinable by receiving expression levels of one or more, preferably three or more, target genes of the signaling pathway, determining an activity level of a signaling pathway associated transcription factor (TF) element in the sample, the TF element controlling transcription of the three or more target genes, the determining being based on evaluating a calibrated mathematical pathway model relating expression levels of the one or more, preferably three or more target genes to the activity level of the signaling pathway, and optionally inferring the activity of the signaling pathway in the epithelial cell based on the determined activity level of the signaling pathway associated TF element. As described herein, the activity level can be directly used as an input to determine the airway abnormality factor and/or risk score, which is also contemplated by the present invention.

The term "activity level" of a TF element, as used herein, denotes the level of activity of the TF element regarding transcription of its target genes.

The calibrated mathematical pathway model may be a probabilistic model, preferably a Bayesian network model, based on conditional probabilities relating the activity level of the signaling pathway associated TF element and the expression levels of the three or more target genes, or the calibrated mathematical pathway model may be based on one or more linear combination(s) of the expression levels of the three or more target genes. For the purposes of the present invention, the calibrated mathematical pathway model is preferably a centroid or a linear model, or a Bayesian network model based on conditional probabilities.

In particular, the determination of the expression level and optionally the inferring of the activity of a signaling pathway in the subject may be performed, for example, by inter alia (i) evaluating a portion of a calibrated probabilistic pathway model, preferably a Bayesian network, representing the cellular signaling pathways for a set of inputs including the expression levels of the three or more target genes of the cellular signaling pathway measured in a sample of the subject, (ii) estimating an activity level in the subject of a signaling pathway associated transcription factor (TF) element, the signaling pathway associated TF element controlling transcription of the three or more target genes of the cellular signaling pathway, the estimating being based on conditional probabilities relating the activity level of the signaling pathway associated TF element and the expression levels of the three or more target genes of the cellular signaling pathway measured in the sample of the subject, and optionally (iii) inferring the activity of the cellular signaling pathway based on the estimated activity level of the signaling pathway associated TF element in the sample of the subject. This is described in detail in the published international patent application WO 2013/011479 A2 ("Assessment of cellular signaling pathway activity using probabilistic modeling of target gene expression.

In an exemplary alternative, the determination of the expression level and optionally the inferring of the activity of a cellular signaling pathway in the subject may be performed by inter alia (i) determining an activity level of a signaling pathway associated transcription factor (TF) element in the sample of the subject, the signaling pathway associated TF element controlling transcription of the three or more target genes of the cellular signaling pathway, the determining being based on evaluating a calibrated mathematical pathway model relating expression levels of the three or more target genes of the cellular signaling pathway to the activity level of the signaling pathway associated TF element, the mathematical pathway model being based on one or more linear combination(s) of expression levels of the three or more target genes, and optionally (ii) inferring the activity of the cellular signaling pathway in the subject based on the determined activity level of the signaling pathway associated TF element in the sample of the subject. This is described in detail in the published international patent application WO 2014/102668 A2 ("Assessment of cellular signaling pathway activity using linear combination(s) of target gene expressions").

Further details regarding the inferring of cellular signaling pathway activity using mathematical modeling of target gene expression can be found in W Verhaegh et al., *2014, supra.*

In an embodiment the signaling pathway measurements are performed using qPCR, multiple qPCR, multiplexed qPCR, ddPCR, RNAseq, RNA expression array or mass spectrometry. For example, a gene expression microarray data, e.g. Affymetrix microarray, or RNA sequencing methods, like an Illumina sequencer, can be used.

The present invention concentrates on the TGF-β pathway, the PI3K-FOXO pathway, the Notch pathway, and/or the HH pathway. According to a preferred embodiment of the present invention, the signaling pathways comprise the TGF-β pathway and one or more of the PI3K-FOXO pathway and the Notch pathway, preferably the TGF-β pathway and at least the PI3K-FOXO pathway.

According to a preferred embodiment of the present invention the risk score is defined such that the indicated risk increases with a decreasing activity of the TGF-β pathway and one or more of an increasing activity of the PI3K pathway, a decreasing activity of the Notch pathway. Similarly, the airway abnormality factor is preferably defined such that the indicated factor reflects an increasing deviation from normal with a decreasing activity of the TGF-β pathway and one or more of an increasing activity of the PI3K pathway, and/or a decreasing activity of the Notch pathway. The increase and/or decrease is preferably a monotonic increase and/or a monotonic decrease. The decreasing activity of the TGF-β pathway and one or more of an increasing activity of the PI3K pathway, a decreasing activity of the Notch pathway is preferably with respect to reference cellular signaling pathway activities.

According to a preferred embodiment of the present invention and the various embodiments thereof, the three or more TGF-β target genes are selected from the group consisting of: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKN1A, CTGF, GADD45B, ID1, IL11, JUNB, SERPINE1, PDGFB, SKIL, SMAD7, SNAI2, and VEGFA, most preferably, from the group consisting of: ANGPTL4, CDC42EP3, ID1, IL11, JUNB, SERPINE1, SKIL, and SMAD7.

According to a preferred embodiment of the present invention and the various embodiments thereof, the three or more PI3K-FOXO target genes are selected from the group consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2, TNFSF10, preferably, from the group consisting of: FBXO32, BCL2L11, SOD2, TNFSF10, BCL6, BTG1, CCNG2, CDKN1B, BNIP3, GADD45A, INSR, and MXI1.

According to a preferred embodiment of the present invention and the various embodiments thereof, the three or more Notch target genes are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target gene(s) are selected from the group consisting of:

DTX1, HES1, HES4, HES5, HEY2, MYC, NRARP, and PTCRA, and one or more Notch target gene(s) are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC.

In this respect, particular reference is made to the sequence listings for the target genes provided with the above-mentioned references as follows:

TGF-β: ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1 and VEGFA (WO 2016/062891, WO 2016/062893);

PI3K-FOXO: AGRP, BCL2L11, BCL6, BNIP3, BTG1, CAT, CAV1, CCND1, CCND2, CCNG2, CDK 1A, CDK 1B, ESR1, FASLG, FBX032, GADD45A, INSR, MXII, NOS3, PCKI, POMC, PPARGCIA, PRDX3, RBL2, SOD2 and TNFSF10 (WO 2015/101635); ATP8A1, BCL2L11, BNIP3, BTGI, ClOorflO, CAT, CBLB, CCNDI, CCND2, CDKNIB, DDB1, DYRK2, ERBB3, EREG, ESRI, EXT1, FASLG, FGFR2, GADD45A, IGF1R, IGFBP1, IGFBP3, INSR, LGMN, MXI1, PPM1D, SEMA3C, SEPP1, SESN1, SLC5A3, SMAD4, SOD2, TLE4, and TNFSF10 (WO 2016/062892, WO 2016/062893); SOD2, BNIP3, MXI1, PCK1, PPARGC1A and CAT (EP16200697.7, supra);

Notch: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9 and TNC (EP 17194288.1, supra);

The set of target genes which are found to best indicate the activity of the respective cellular signaling pathway, based on microarray/RNA sequencing based investigation using, e.g., the Bayesian model or the (pseudo-)linear model, can be translated into for example a multiplex quantitative PCR assay or dedicated microarray biochips to be performed on a sample of a subject. A selection of the gene sequence as described herein can be used to select for example a primer-probe set for RT-PCR or oligonucleotides for microarray development. To develop such an FDA-approved test for pathway activity and risk score determination, development of a standardized test kit is required, which needs to be clinically validated in clinical trials to obtain regulatory approval.

In accordance with a third aspect, the present invention relates to an apparatus for determining an airway abnormality factor indicating whether a subject has abnormal airway epithelium or a risk score that indicates a risk that a subject having abnormal airway epithelium will develop an airway cancer comprising a digital processor configured to perform the method of the first and/or second aspect of the present invention and the various embodiments thereof. Accordingly the invention relates to an apparatus cancer comprising a digital processor configured to perform the method of the first and/or second aspect of the present invention and the various embodiments thereof.

In accordance with a fourth aspect, the present invention relates to a non-transitory storage medium for determining an airway abnormality factor indicating whether a subject has abnormal airway epithelium or a risk score that indicates a risk that a subject having abnormal airway epithelium will develop an airway cancer storing instructions that are executable by a digital processing device to perform the method of the first and/or second aspect of the present invention and the various embodiments thereof. The non-transitory storage medium may be a computer-readable storage medium, such as a hard drive or other magnetic storage medium, an optical disk or other optical storage medium, a random access memory (RAM), read only memory (ROM), flash memory, or other electronic storage medium, a network server, or so forth. The digital processing device may be a handheld device (e.g., a personal data assistant or smartphone), a notebook computer, a desktop computer, a tablet computer or device, a remote network server, or so forth. Accordingly the invention relates to a non-transitory storage medium storing instructions that are executable by a digital processing device to perform the method of the first and/or second aspect of the present invention and the various embodiments thereof.

In accordance with a fifth aspect, the present invention relates to a computer program for determining an airway abnormality factor indicating whether a subject has abnormal airway epithelium or a risk score that indicates a risk that a subject having abnormal airway epithelium will develop an airway cancer comprising program code means for causing a digital processing device to perform a method according to the first and/or second aspect of the present invention and the various embodiments thereof, when the computer program is run on the digital processing device. The computer program may be stored/distributed on a suitable medium, such as an optical storage medium or a solid-state medium, supplied together with or as part of other hardware, but may also be distributed in other forms, such as via the Internet or other wired or wireless telecommunication systems. Accordingly the invention relates to a computer program comprising program code means for causing a digital processing device to perform a method according to the first and/or second aspect of the present invention and the various embodiments thereof, when the computer program is run on the digital processing device.

In accordance with a sixth aspect, the present invention relates to a kit for determining an airway abnormality factor indicating whether a subject has abnormal airway epithelium or a risk score that indicates a risk that a subject having abnormal airway epithelium will develop an airway cancer, the kit comprising components for determining the expression levels of at least three target genes of a TGF-β cellular signaling pathway, at least three target genes of a PI3K-FOXO cellular signaling pathway, at least three target genes of a Notch cellular signaling pathway and/or at least three target genes of a HH cellular signaling pathway. Preferably the kit comprises components for determining the expression levels of at least three target genes of a TGF-β cellular signaling pathway, at least three target genes of a PI3K-FOXO cellular signaling pathway, and/or at least three target genes of a Notch cellular signaling pathway. In a preferred embodiment of the kit according to the invention, the three or more TGF-β target genes are selected from the group consisting of: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKN1A, CTGF, GADD45B, ID1, IL11, JUNB, SERPINE1, PDGFB, SKIL, SMAD7, SNAI2, and VEGFA, most preferably, from the group consisting of: ANGPTL4, CDC42EP3, ID1, IL11, JUNB, SERPINE1, SKIL, and SMAD7, or wherein the three or more TGF-β target genes are selected from the group consisting of: CDC42EP3, GADD45B, HMGA2, ID1, JUNB, OVAL1, VEGFA, SGK1, and

the three or more PI3K-FOXO target genes are selected from the group consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2, TNFSF10, preferably, from the group consisting of: FBXO32, BCL2L11, SOD2, TNFSF10, BCL6, BTG1, CCNG2, CDKN1B, BNIP3, GADD45A, INSR, and MXI1, and the three or more Notch target genes are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target gene(s) are selected from the group consisting of: DTX1, HES1, HES4, HES5, HEY2, MYC, NRARP, and PTCRA, and one or more Notch target gene(s) are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TN.

Another aspect of the invention pertains to use of components for determining the expression levels of at least three target genes of a TGF-β cellular signaling pathway, at least three target genes of a PI3K-FOXO cellular signaling pathway, at least three target genes of a Notch cellular signaling pathway and/or at least three target genes of a HH cellular signaling pathway for the manufacture of a kit for determining an airway abnormality factor indicating whether a subject has abnormal airway epithelium or a risk score that indicates a risk that a subject having abnormal airway epithelium will develop an airway cancer.

The kit is in particular a quantitative kit, i.e. allows quantification of the expression levels.

The kit may comprises one or more components or means for measuring (in particular quantifying) the expression levels of the target genes selected from the group consisting of: a DNA array chip, an oligonucleotide array chip, a protein array chip, an antibody, a plurality of probes, for example, labeled probes, a set of RNA reverse-transcriptase sequencing components, and/or RNA or DNA, including cDNA, amplification primers. In a preferred embodiment, the kit is selected from the group consisting of qPCR, multiple qPCR, multiplexed qPCR, ddPCR, RNAseq, RNA expression array and mass spectrometry. In an embodiment, the kit includes a set of labeled probes directed to a portion of an mRNA or cDNA sequence of the target genes as described herein. In an embodiment, the kit includes a set of primers and probes directed to a portion of an mRNA or cDNA sequence of the target genes. In an embodiment, the labeled probes are contained in a standardized 96-well plate. In an embodiment, the kit further includes primers or probes directed to a set of reference genes. Such reference genes can be, for example, constitutively expressed genes useful in normalizing or standardizing expression levels of the target gene expression levels described herein.

Therefore, in an embodiment the invention relates to a kit, the kit comprising: components for determining the expression levels of at least three target genes of a TGF-β cellular signaling pathway, at least three target genes of a PI3K-FOXO cellular signaling pathway, and/or at least three target genes of a Notch cellular signaling pathway, wherein:

the three or more TGF-β target genes, for example 3, 4, 5, 6, 7, 8, 9, 10, 11, 12, or more genes, are selected from the group consisting of: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, the three or more TGF-β target genes, for example 3, 4, 5, 6, 7, 8, 9, 10, 11, 12, 13, or 14 genes, are selected from the group consisting of: ANGPTL4, CDC42EP3, CDKN1A, CTGF, GADD45B, ID1, IL11, JUNB, SERPINE1, PDGFB, SKIL, SMAD7, SNAI2, and VEGFA, most preferably, the three or more TGF-β target genes, for example 3, 4, 5, 6, 7 or 8 genes, are selected from the group consisting of: ANGPTL4, CDC42EP3, ID1, IL11, JUNB, SERPINE1, SKIL, and SMAD7, alternatively wherein the three or more TGF-β target genes, for example 3, 4, 5, 6, 7 or 8 genes, are selected from the group consisting of: CDC42EP3, GADD45B, HMGA2, ID1, JUNB, OVAL1, VEGFA, SGK1 and/or the three or more PI3K-FOXO target genes, for example 3, 4, 5, 6, 7, 8, 9, 10, 11, 12, or more genes, are selected from the group consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2, TNFSF10, preferably the three or more PI3K-FOXO target genes, for example 3, 4, 5, 6, 7, 8, 9, 10, 11, 12 or 13 genes, are selected from the group consisting of: FBXO32, BCL2L11, SOD2, TNFSF10, BCL6, BTG1, CCNG2, CDKN1B, BNIP3, GADD45A, INSR, and MXI1, and/or

the three or more Notch target genes, for example 3, 4, 5, 6, 7, 8, 9, 10, 11, 12, or more genes, are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target genes, for examle 2, 3, 4, 5, 6, 7 or 8 genes, are selected from the group consisting of: DTX1, HES1, HES4, HES5, HEY2, MYC, NRARP, and PTCRA, and one or more Notch target gene(s), for examples 1, 2, 3, 4, 5, 6, 7, 8, 9, 10, 11, 12, or more genes, are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC. Preferably the kit is for, or is suitable for determining an airway abnormality factor indicating whether a subject has abnormal airway epithelium or a risk score that indicates a risk that a subject having abnormal airway epithelium will develop an airway cancer

In some embodiments, the kit is not a microarray for determining expression levels of thousands of target genes. For example, the kit of the present invention may include components for determining expression levels of not more than 1000 target genes, not more than 700 target genes, not more than 500 target genes, not more than 200 target genes, not more than 100 target genes, in addition to the components required for the specific target genes disclosed herein.

The kit may further comprise the apparatus of the third aspect, the non-transitory storage medium of the fourth aspect, or the computer program of the fifth aspect.

In accordance with a seventh aspect, the present invention relates to a kit for use in a method of diagnosing or prognosticating whether a subject has abnormal airway epithelium or whether a subject having abnormal airway epithelium will develop an airway cancer, the kit comprising components for determining the expression levels of at least three target genes of a TGF-β cellular signaling pathway, at least three target genes of a PI3K-FOXO cellular signaling pathway, at least three target genes of a Notch cellular signaling pathway and/or at least three target genes of a HH cellular signaling pathway. According to a preferred embodiment the method of diagnosing or prognosticating comprises extracting an epithelial cell sample from an airway of the subject, and subjecting the extracted epithelial cell sample to a method according to the first and/or second aspect of the present invention.

Another aspect of the present invention is a method of diagnosing or prognosticating whether a subject has abnormal airway epithelium or whether a subject having abnormal airway epithelium will develop an airway cancer, the method comprising providing an epithelial cell sample of an airway of the subject or extracting an epithelial cell sample from an airway of the subject, and subjecting the provided or extracted epithelial cell sample to a method according to the first and/or second aspect of the present invention.

Therefore, the invention further relates to a method for *in vitro* or *ex vivo* diagnosing or prognosticating whether a subject has abnormal airway epithelium or whether a subject having abnormal airway epithelium will develop an airway cancer using a kit, the kit comprising components for determining the expression levels of at least three target genes of a TGF-β cellular signaling pathway, at least three target genes of a PI3K-FOXO cellular signaling pathway, at least three target genes of a Notch cellular signaling pathway, the method comprising: determining an airway abnormality factor indicating whether the subject has abnormal airway epithelium based on a combination of activities of cellular signaling pathways in an epithelial cell sample derived from an airway of the subject, wherein the cellular signaling pathways comprise two or more cellular signaling pathways selected from the group consisting of a TGF-β pathway, a PI3K-FOXO pathway, and a Notch pathway, wherein the determining of the signaling pathway abnormality factor is further based on a reference activity of the respective cellular signaling pathway, wherein the reference activity reflects activity of the respective cellular signaling pathway found in airway epithelium of healthy subjects, and wherein the determining of the airway abnormality factor comprises: determining a signaling pathway abnormality factor for each of the respective cellular signaling pathways based on the activity of the respective cellular signaling pathway in the epithelial cell sample and determining the airway abnormality factor based on a combination of the determined cellular signaling pathway abnormality factors, wherein a quantitative measurement of signal transduction pathway activity in epithelial cells obtained from upper or lower airways is based on inferring activity of a signal transduction pathway from measurements of mRNA levels of three or more validated direct target genes of the transcription factor associated with the respective signaling pathway. Preferably in said method for *in vitro* or *ex vivo* diagnosing or prognosticating an epithelial cell sample of an airway of the subject is provided, or an extracted epithelial cell sample from an airway of the subject is provided, and subjecting the provided or extracted epithelial cell sample to a method according to the first and/or second aspect of the present invention. Preferably said method further comprises providing an epithelial cell sample of an airway of the subject or extracting an epithelial cell sample from an airway of the subject.

Preferably, in said method for *in vitro* or *ex vivo* diagnosing or prognosticating, said components for determining the expression levels of at least three target genes of a TGF-β cellular signaling pathway, at least three target genes of a PI3K-FOXO cellular signaling pathway, at least three target genes of a Notch cellular signaling pathway, comprise the three or more TGF-β target genes, for example 3, 4, 5, 6, 7, 8, 9, 10, 11, 12, or more genes, are selected from the group consisting of: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, the three or more TGF-β target genes, for example 3, 4, 5, 6, 7, 8, 9, 10, 11, 12, 13, or 14 genes, are selected from the group consisting of: ANGPTL4, CDC42EP3, CDKN1A, CTGF, GADD45B, ID1, IL11, JUNB, SERPINE1, PDGFB, SKIL, SMAD7, SNAI2, and VEGFA, most preferably, the three or more TGF-β target genes, for example 3, 4, 5, 6, 7 or 8 genes, are selected from the group consisting of: ANGPTL4, CDC42EP3, ID1, IL11, JUNB, SERPINE1, SKIL, and SMAD7, alternatively wherein the three or more TGF-β target genes, for example 3, 4, 5, 6, 7 or 8 genes, are selected from the group consisting of: CDC42EP3, GADD45B, HMGA2, ID1, JUNB, OVAL1, VEGFA, SGK1, and the three or more PI3K-FOXO target genes, for example 3, 4, 5, 6, 7, 8, 9, 10, 11, 12, or more genes, are selected from the group consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2, TNFSF10, preferably the three or more PI3K-FOXO target genes, for example 3, 4, 5, 6, 7, 8, 9, 10, 11, 12 or 13 genes, are selected from the group consisting of: FBXO32, BCL2L11, SOD2, TNFSF10, BCL6, BTG1, CCNG2, CDKN1B, BNIP3, GADD45A, INSR, and MXI1, and the three or more Notch target genes, for example 3, 4, 5, 6, 7, 8, 9, 10, 11, 12, or more genes, are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target genes, for examle 2, 3, 4, 5, 6, 7 or 8 genes, are selected from the group consisting of: DTX1, HES1, HES4, HES5, HEY2, MYC, NRARP, and PTCRA, and one or more Notch target gene(s), for examples 1, 2, 3, 4, 5, 6, 7, 8, 9, 10, 11, 12, or more genes, are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC. Preferably said kit is the kit as described in the kit according to the sixth apsect of the invention.

The method for *in vitro* or *ex vivo* diagnosing or prognosticating whether a subject has abnormal airway epithelium or whether a subject having abnormal airway epithelium will develop an airway cancer according to the invention further comprises determining an airway abnormality factor indicating whether the subject has abnormal airway epithelium based on a combination of activities of cellular signaling pathways in an epithelial cell sample derived from an airway of the subject, wherein the cellular signaling pathways comprise two or more cellular signaling pathways selected from the group consisting of a TGF-β pathway, a PI3K-FOXO pathway, and a Notch pathway, wherein the determining of the signaling pathway abnormality factor is further based on a reference activity of the respective cellular signaling pathway, wherein the reference activity reflects activity of the respective cellular signaling pathway found in airway epithelium of healthy subjects.

In accordance with a eights aspect, the present invention relates to a kit comprising components for determining the expression levels of at least three target genes of a TGF-β cellular signaling pathway, at least three target genes of a PI3K-FOXO cellular signaling pathway, at least three target genes of a Notch cellular signaling pathway. Preferably the kit is for or is suitable for determining a risk score that indicates a that a subject has abnormal airway epithelium or whether a subject having abnormal airway epithelium will develop an airway cancer.

One advantage of the present invention resides in a clinical decision support (CDS) system that is adapted to provide clinical recommendations, e.g., by deciding a treatment for a subject, based on a combination of pathway activities as described herein, as indicated by an airway abnormality factor and/or a risk score that is determined based on the combination of the pathway activities.

Another advantage resides in a CDS system that is adapted to assign a subject to at least one of a plurality of risk groups associated with different risks that the subject will develop an airway cancer, as indicated by an airway abnormality factor and/or a risk score that is determined based on a combination of activities of two or more cellular signaling pathways as described herein.

Another advantage resides in combining a risk score that indicates a risk that a subject will develop an airway cancer and that is determined based on a combination of activities of two or more cellular signaling pathways as described herein with one or more additional risk scores obtained from one or more additional prognostic tests.

The present invention as described herein can, e.g., also advantageously be used in connection with

prediction whether airway epithelium is pre-malignant, and/or
prediction whether a person has a high risk at development of airway cancer (as defined herein), and/or
prediction whether a person has a high risk at development of lung cancer, and/or
prediction whether a person has a high risk at development of squamous lung cancer, and/or
prediction whether a person has a high risk at development of lung adenocarcinoma, and/or
prediction whether a person can benefit from a local therapy to prevent development of cancer, and/or
prediction whether a patient has lung cancer, and/or

prognosis and/or prediction based on a combination of activities of two or more cellular signaling pathways, and/or prediction of drug efficacy of e.g. chemotherapy and/or hormonal treatment based on a combination of activities of two or more cellular signaling pathways, and/or monitoring of drug efficacy based on a combination of activities of two or more cellular signaling pathways, and/or

deciding on a frequency of monitoring or, more particularly, on a frequency of therapy response monitoring based a combination of activities of two or more cellular signaling pathways, and/or

drug development based a combination of activities of two or more cellular signaling pathways, and/or

assay development based on a combination of activities of two or more cellular signaling pathways, and/or

prediction whether a person is at risk of developing invasive airway cancer, and/or

prediction whether a person is at risk of disease progression, and/or prediction or diagnosis whether a person has reduced risk after treatment (e.g. chemoprevention), and/or

prediction whether a nodule or abnormality detected with an imaging modality, such as a CT scan, is more likely to be malignant or benign and/or

cancer staging based on a combination of activities of two or more cellular signaling pathways,

wherein in each case, the cellular signaling pathways comprise an TGF-$\beta$ pathway, an PI3K-FOXO pathway, and/or a Notch pathway.

Further advantages will be apparent to those of ordinary skill in the art upon reading and understanding the attached figures, the following description and, in particular, upon reading the detailed examples provided herein below.

It shall be understood that the methods of the first and second aspect, the apparatus of the third aspect, the non-transitory storage medium of fourth aspect, the computer program of the fifth aspect, the kits of the sixth, seventh and eighth aspects have similar and/or identical preferred embodiments, in particular, as defined in the dependent claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Calculations like the determination of the risk score performed by one or several units or devices can be performed by any other number of units or devices.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

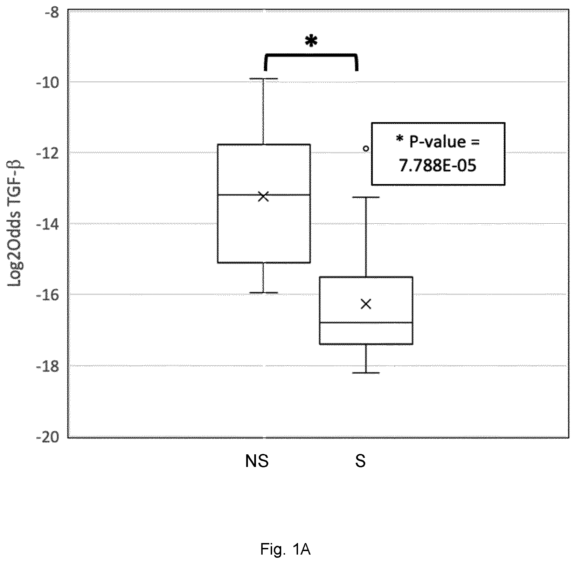

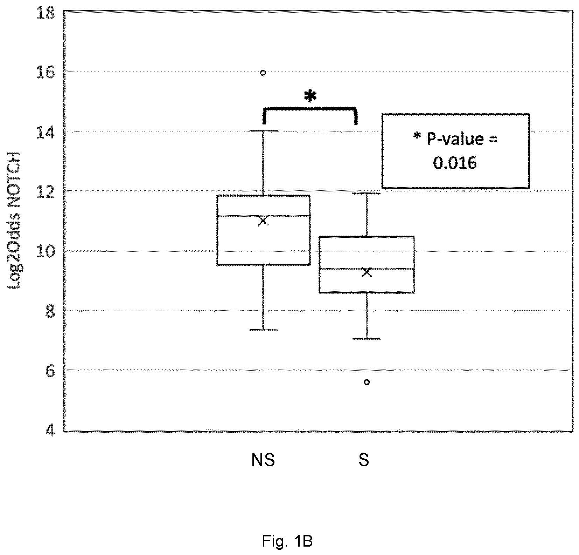

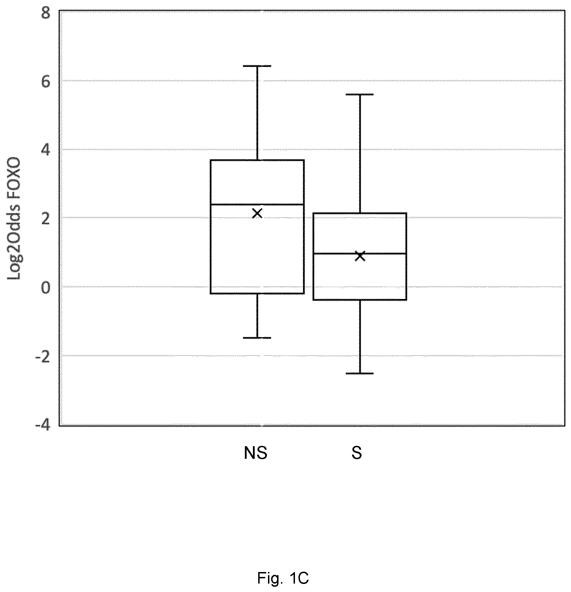

Fig. 1 shows exemplarily results for the determination of signaling pathway activities measured in normal small airway epithelial cells obtained by brushing from non-smokers (n=13) (left box, labelled "NS") and normal small airway epithelial cells obtained from heavy smokers (n=18) (right box, labelled "S") (Fig. 1A. TGF-b; Fig. 1B. Notch; Fig. 1C: PI3K-FOXO). Log2odds pathway scores are shown.

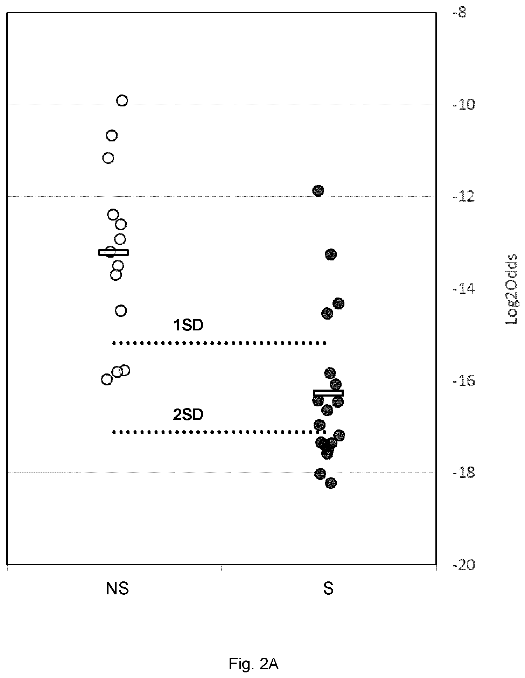

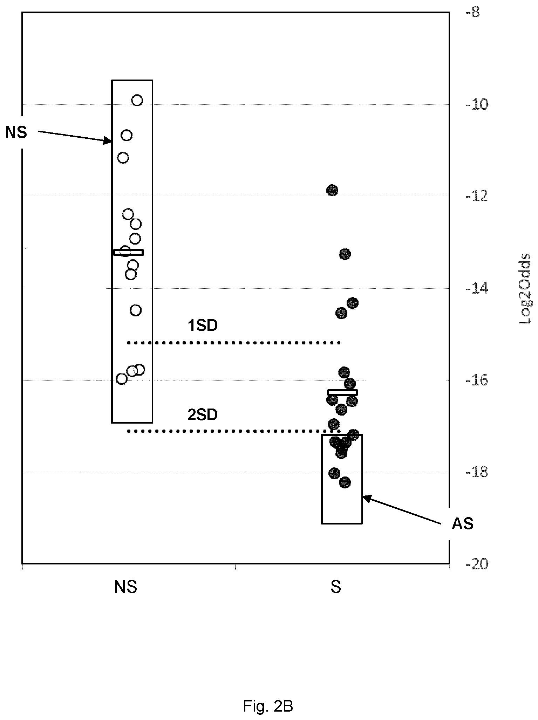

Fig. 2 shows exemplarily calculation of a threshold for abnormal pathway activity in airway epithelial cells using TGF-$\beta$ pathway activity using dataset GSE10006. Here depicted are the pathway activity scores for each sample in the dataset, separately for "non-smokers" and "smokers". For both groups the mean for the pathway activity score is calculated (shown in Fig. 2 as small rectangular), as well as the standard deviation (SD). The threshold for abnormal low TGF-$\beta$ activity is set as mean pathway activity score minus 1SD, or alternatively mean minus 2SD, of TGF-$\beta$ pathway scores of healthy non-smokers for normal small airways, and shown in Fig. 2 as horizontal dotted lines respectively labelled with "1SD" and "2SD". A TGF-$\beta$ pathway score below this level only occurs in 15.8 % of healthy non-smokers if 1SD is taken as the threshold, and only 2.2 % of that population if 2SD is taken at the lower threshold. A value lower than this threshold is considered to be associated with an in-creasing risk (depending on 1 SD or 2SD threshold) of being derived from abnormal air-way epithelium. For development of the here described centroid-computational method, a sample pathway analysis result below the defined 2SD threshold is considered abnormal. In this case abnormal pathway activity is characterized by loss of tumor suppressive TGF-$\beta$ activity. In the smoker group of this dataset, it is expected that not all, but only a subgroup of smokers will have an abnormal airway epithelium. This subgroup can be identified by applying the TGF-$\beta$ pathway activity threshold for normal epithelium to this group. Indeed, a relatively large number of samples had a TGF-$\beta$ pathway activity below the respective 1SD and 2SD threshold (Figure 2B). For subsequent calibration of the centroid computational model the samples that have a TGF-$\beta$

pathway score below 2SD of normal are considered as calibration set for abnormal samples. On the other hand, the non-smoker samples that have a TGF-β pathway score higher than the 2SD threshold constitute a calibration set for normal samples. For the linear model, as an example, the 1SD threshold is taken as threshold below which a pathway activity score is considered as abnormally low. This can be done in a corresponding manner for the other signaling pathways. Fig. 2B additionally indicates the calibration data in quadrangles. The left quadrangle indicates the calibration set for the normal samples "NS". The right quadrangle indicates the calibration set for the abnormal samples "AS".

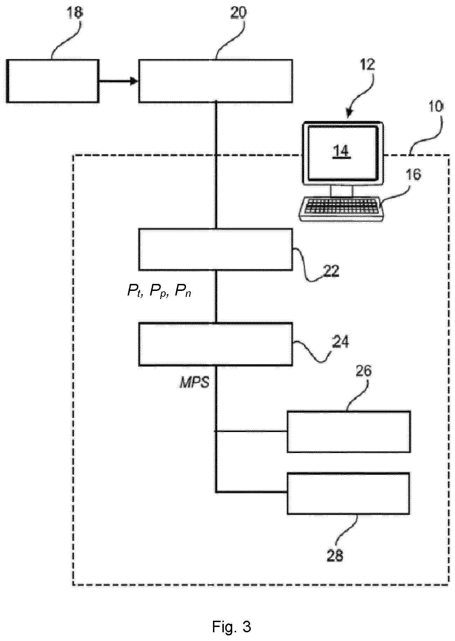

Fig. 3 diagrammatically shows a clinical decision support (CDS) system configured to determine a risk score that indicates a risk that a subject will develop an airway cancer, as disclosed herein.

## DETAILED DESCRIPTION OF EMBODIMENTS

The following embodiments merely illustrate particularly preferred methods and selected aspects in connection therewith. The teaching provided therein may be used for constructing several tests and/or kits. The following examples are not to be construed as limiting the scope of the present invention.

The present invention relates to a method that can identify individuals, especially smokers, who have abnormal/pre-malignant changes in the airway epithelium and/or are at increased risk for developing lung cancer. Measurement of signal transduction pathway activity can identify abnormal signal transduction pathway activity indicative of abnormal airway epithelium, and increased likelihood of pre-malignant change. In accordance with the present invention, activity of two or more pathways are determined and translated into an airway abnormality factor, in the following also denoted as (Small/Large) Airway pathway abnormality score, (S/L)APAS, that identify individuals with abnormal airway epithelium, with potentially increased risk at developing lung cancer.

The present invention provides a risk score and or an airway epithelial abnormality factor/score indicative for the presence of premalignant changes in epithelial cells derived from airways, based on combined activities of the PI3K-FOXO pathway, the Notch, HH and/or TGF-β pathways, and interpretation of the pathway results using a computational model which provides a risk score and/or airway epithelial abnormality factor. Analyzing epithelial cells derived from large or small airways can be used to early detect abnormalities that may indicate a higher risk at developing lung cancer. The risk score and/or airway epithelial abnormality factor can also be used to stratify patient for close monitoring or aid in selection of treatments directed at returning these pathways to their non-pathological state to reverse the premalignant alterations.

Lung cancer develops in the larger airway (bronchi branching off the trachea) or small airway epithelium. Using techniques like airway brushing, collection of nasal cells (nasal swabs) or broncho-alveolar lavage, epithelial cells can be obtained for molecular analysis from upper and lower airways in a relatively non-invasive manner. This provides a potential means to identify individuals, especially smokers, that have these early proliferative changes in their airway epithelium and are at increased risk for developing lung cancer.

Signal transduction pathway analysis enables quantitative measurement of signal transduction pathway activity in epithelial cells obtained from upper or lower airways, for example by broncho-alveolar lavage, brushing, or biopsy, and is based on inferring activity of a signal transduction pathway from measurements of mRNA levels of validated direct target genes of the transcription factor associated with the respective signaling pathway (see for example W Verhaegh et al., 2014, *supra;* W Verhaegh, A van de Stolpe, 2014, *supra)*. The determining of the activity of one or more pathways, the combination of multiple pathway activities and applications thereof may be performed as described above. The models have been biologically validated for ER, AR, PI3K-FOXO, HH, Notch, TGF-β, Wnt, NFkB and STAT1/2 and STAT3 pathways on several cell types, including epithelial cells.

The present invention concentrates on the TGF-β pathway, the PI3K-FOXO pathway, the Notch pathway, and/or the HH pathway.

TGF-β is involved in regulation of cell proliferation, differentiation, immune cell activity, the cellular microenvironment and other cellular processes. In normal and premalignant cells TGF-β exerts a tumor suppressive function. However, in the progression to cancerous cells, the tumor suppressive effects may be lost by receptor-inactivating mutations or selective loss of the suppressive arm of the pathway (Massague et al., Cell, 2008, 134(2): 215-230). Tobacco exposure can reduce TGF-β-mediated growth inhibition and apoptosis, which is indicative for the smoking promotes tumorigenicity (Samanta et al., Cancer Prev Res, 2012, 5(3): 453-63).

The PI3K-FOXO pathway is commonly hyper-activated in various types of cancer. Tumors are potentially sensitive to PI3K-FOXO pathway inhibitors but reliable diagnostic tests assessing functional PI3K-FOXO activity lack. As the PI3K-FOXO pathway negatively regulates the tumor suppressive FOXO transcription factors, FOXO target gene expression is inversely correlated to PI3K activity (on the premise that there is no oxidative stress (van Ooijen, 2018, *supra*). It has been shown that FOXO3 deficiency leads to increased susceptibility to cigarette smoke-induced inflammation, airspace enlargement, and COPD (Hwang et al, J Immunol, 2011, 187(2): 987-998). Levels of FOXO3 are significantly decreased in lungs of smokers and patients with chronic obstructive pulmonary disease (COPD).

Notch signaling is involved in regulation of cell proliferation, differentiation and apoptosis. The tumor suppressive versus tumor promoting function of the different isoforms of Notch is depending on the cellular and environmental context. In one tumor for example Notch1 has been described to have an oncogenic role in promotion of tumor initiation and Notch2 a tumor suppressive role (Zou et al, Oncology Letters, 2018, 15: 3415-3421). However, this can be different in different tumor types, e.g. in badder cancer both NOTCH1 and NOTCH2 are tumor suppressive (Cancer Discov, 2014, 4(11):1252) Expression levels of the different isoforms different between the various histological subtypes of lung cancer (Chen et al., Journal of Cancer, 2017, 8(7):1292-1300). In one type of lung cancer, SCLC, the tumor suppressive function of the Notch pathway is lost, and this even provides a therapeutic target, drugs being developed to increase Notch pathway activity in patients with SCLC (Nat Rev Clin Oncol, 2017, 14(9): 549-561). The Notch pathway is down-regulated in the airway epithelium of healthy smokers and smokers with chronic obstructive pulmonary disease, implying that this pathway may be important in repair of smoking-induced injury (Tilley et al., Am J Respir Crit Care Med, 2009, 179(6):457-66).

**Signaling pathway analysis of Affymetrix U133 Plus2.0 expression microarray datasets (GEO, public datset) with data from smokers and non-smokers** In the GEO dataset database (https://www.ncbi.nlm.nih.gov/gds/) an Affymetrix Plus2.0 publicly available clinical study was identified containing Affymetrix data from epithelial airway cells from non-smokers, and light and heavy tobacco smokers.

Dataset GSE10006. The investigated groups were: large airways of smokers (n=9); large airways of non-smokers (n=20); small airways of smokers (n=13); small airways of non-smokers (n=18), and as an independent dataset for validation purposes the COPD patient data. Results for analysis of TGFbeta, NOTCH pathway and FOXO transcription factor activity are shown for normal small airway epithelium of non-smokers and smokers, in Figs. 1A to 1C. Expression levels of the herein disclosed target genes were gathered from the datasets for each of the TGF-$\beta$, PI3K-FOXO, and Notch pathways. Subsequently, signaling pathway activity was determined as described herein, and compared between non-smokers and smokers. Pathway activities are indicated on a log2 odds scale, and significant differences (Rank Wilcoxon test) are indicated.

Using this approach, it was found that TGF-$\beta$ and Notch pathway activity are lost in a subpopulation of heavy smokers (cf. Figs. 1A and 1B), while activity of the FOXO transcription factor may be lost in a subgroup of smokers in which the PI3K growth factor pathway has been activated (cf. Fig. 1C). This means, in airway epithelial cells from a subgroup of heavy smokers, compared to non-smokers, there is a characteristic pathway activity profile: Loss of the tumor suppressive effect of TGF-$\beta$ pathway and Notch pathway activity, associated with increased activity of the proliferative PI3K pathway. This indicates abnormal proliferation and loss of tumor suppressive activity of Notch and TGF-$\beta$ pathways in a subgroup of the heavy smoker population. The subjects of this subgroup are probably at high risk for development of a form of lung cancer. When these abnormalities are found in cells derived from the upper airways, they are likely at higher risk to develop cancers that typically arise here, like squamous lung cancer; and when present in lower airway epithelial cells the subject is likely to be at higher risk to develop adenocarcinoma, which typically arises in the lower airways.

It was also confirmed that epithelial cells from the trachea (upper airway) show similar pathway activity abnormalities as found in lower airway epithelial cells, and thus can likely be used as surrogate sample to provide an epithelial abnormality score, and cancer risk score (data not shown). Epithelial cells from the trachea are easier to obtain, and the sampling is less invasive.

Using dataset GSE19722 it was further found that the present invention is applicable to stem/progenitor cells of epithelial cells (Dataset GSE19722. REF: Shaykhiev R, Wang R, Zwick RK, Hackett NR et al. Airway basal cells of healthy smokers express an embryonic stem cell signature relevant to lung cancer. Stem Cells 2013 Sep;31(9):1992-2002). In particular, cultured basal cells (BCs), which constitute the stem/progenitor cells needed for regeneration of damaged epithelium may therefore be used as a surrogate sample for primary, non-cultured, epithelial cell sample. The reduced FOXO transcription factor activity in basal cells from smokers as compared to non-smokers is indicative of increased PI3K pathway activity (cf. Table 1).

Table 1: Analysis of stem/progenitor cells of epithelial cells using dataset GSE19722. Small airway epithelial cells were collected via flexible bronchoscopy and cultured; after a week RNA were extracted for Affymetrix Microarray analysis. Log2odds values for FOXO activity are indicated.

| array | sample | FOXO log2odds |
| --- | --- | --- |
| GSM492607 | large airways, basal cell culture non-smoker 118 | 4,399 |
| GSM492608 | large airways, basal cell culture non-smoker 165 | -2.217 |
| GSM492609 | large airways, basal cell culture non-smoker 169 | 1.320 |
| GSM492610 | large airways, basal cell culture non-smoker 194 | 0.558 |
| GSM492612 | large airways, basal cell culture smoker 328 | -5.630 |
| GSM492613 | large airways, basal cell culture smoker 350 | -4.258 |

(continued)

| array | sample | FOXO log2odds |
|---|---|---|
| GSM492614 | large airways, basal cell culture smoker 353 | 2.842 |
| GSM492615 | large airways, basal cell culture smoker 359 | -4.444 |

Earliest damage to the airway epithelium associated with smoking is reflected in hyperplasia of BCs, caused by the increased activity of the PI3K pathway, as identified by the herein described Philips pathway analysis. In this case, BCs were obtained by bronchoscopy from non-smokers and smokers, and cultured for a week on collagen prior to analysis. Under these conditions, PI3K-FOXO activity increased (not significant) in the cultured BCs from smokers, indicating gain of PI3K-FOXO pathway activity (decreased FOXO activity) (cf. Table 1). Abnormal PI3K pathway activity in this cell type correlates with abnormal pathway activity in the epithelial cell type, suggesting that PI3K pathway activity seen in the epithelial cells originate in the basal cells. This experiment shows that a sample from BCs can in principle also be used to measure the abnormality of the epithelial cells.

## Definition of normal pathway activity

Based on pathway activity in healthy non-smoking subjects (GSE10006), a reference pathway activity was defined for normal small airway epithelium. Pathway activity measured in a patient sample was considered abnormal when the measured pathway activity was more than 1 standard deviation (>1SD), or alternatively more than 2 standard deviations (>2SD), below the mean of the normal pathway activity for Notch, TGF-$\beta$ pathways and FOXO transcription factor activity. The determined means and standard deviations for the respective pathways are shown in Table 2.

Table 2. Mean values and standard deviations of the activity of TGF-$\beta$, NOTCH pathway and FOXO transcription factor.

| Pathway | Mean | Standard deviation (SD) |
|---|---|---|
| TGF-b | -13.23 | 1.94 |
| NOTCH | 11.01 | 2.22 |
| PI3K-FOXO | 2.13 | 2.27 |

Based on these values lower thresholds of pathway activities were calculated, below which the pathway activity is considered abnormal in airway epithelial cells:

-15.17 (1SD) and -17.12 (2SD) for lower threshold TGF-$\beta$ activity;
8.79 (1SD) and 6.57 (2SD) for lower threshold NOTCH activity;
-0.14 (1SD) and -2.40 (2SD) for lower threshold PI3K-FOXO activity.

The thus calculated threshold values of the TGF-$\beta$ pathway activity are indicated in Figs. 2A and 2B as horizontal dashed lines.

## Development and calibration of computational models

Due to the fact that individual pathway activities show variability between patient samples, a computational model is advantageously employed to interpret multiple pathway activities and provide a probability that the analyzed epithelial cell sample is not normal, and presumed to present an indicator of a pre-malignant state. For calibration of the models, Affymetrix U133Plus2.0 data from small airway epithelium from healthy smoker and non-smoker from dataset GSE10006 was used; for validation purposes from the same dataset the independent data from samples of smokers with COPD were used.

The model can be a linear, a centroid, a Bayesian model or another model, as described herein. Models can be developed for lower and for higher airway epithelial cells separately, which could provide abnormality scores indicating risk at development of different lung cancer types.

In this example, a centroid model and a linear model was used. However, as will be understood by the skilled person a Bayesian model or the like can be likewise used.

*Centroid model*

An exemplary computational model was developed which uses the pathway activities of PI3K, TGF-β, and Notch measured in epithelial cells obtained from lower (small) airways using brushing as input to calculate a centroid-model (small/large) airway pathway abnormality score.

Selection of samples for calibrating the centroid computational model was as follows (cf. Fig. 2B):

Selection of "normal samples" based on healthy/ normal small airways (non-smoker) above the 2 x SD threshold of the non-smoker data

Selection of "abnormal samples" is based on healthy/normal small airway (smoker) below the 2 x SD threshold of the non-smoker data

The model was calibrated using combined TGF-β and FOXO activities (cf. Table 3), or combined TGF-β and FOXO and Notch activities (cf. Table 4). Before using microarray data, extensive quality control (QC) was performed on Affymetrix data from each individual sample as described elsewhere (A van de Stolpe et al., 2019, *supra*). Only samples that passed QC were used for further analysis.

Table 3. Calibration data of a model based on combined TGF-β, NOTCH and FOXO activities using GSE10006 dataset. Each line represents an individual sample. Sample "NS" denotes a sample from a non-smoker (calibration normal samples). Sample "AS" denotes a sample of a smoker (calibration abnormal samples). All samples passed quality control.

| array (GSM...) | Sample | pathway activity (log2odds scores) | | | Distance of sample to | | Detected as |
|---|---|---|---|---|---|---|---|
| | | FOXO | TGF-β | NOTCH | calibration normal | calibration abnormal | |
| 252856 | NS | 4,449 | -12,602 | 9,692 | 1,954 | 5,970 | Normal |
| 252857 | NS | 1,184 | -12,918 | 9,123 | 1,925 | 4,521 | Normal |
| 252858 | NS | 6,422 | -14,465 | 11,565 | 4,139 | 6,697 | Normal |
| 252859 | NS | 2,371 | -15,759 | 9,876 | 2,599 | 2,343 | Abnormal |
| 252860 | NS | -0,229 | -12,387 | 12,083 | 3,527 | 6,070 | Normal |
| 252861 | NS | 2,368 | -13,185 | 9,968 | 0,454 | 4,572 | Normal |
| 252862 | NS | -0,160 | -15,790 | 11,167 | 3,943 | 3,002 | Abnormal |
| 252863 | NS | 3,565 | -13,490 | 11,621 | 1,620 | 5,401 | Normal |
| 252864 | NS | 2,695 | -13,690 | 11,304 | 1,124 | 4,729 | Normal |
| 252865 | NS | 3,789 | -11,151 | 9,386 | 2,503 | 6,879 | Normal |
| 252866 | NS | 3,143 | -9,907 | 7,354 | 4,442 | 7,980 | Normal |
| 252871 | AS | -0,753 | -17,177 | 7,682 | 5,861 | 2,192 | Abnormal |
| 252874 | AS | 1,362 | -17,486 | 11,932 | 4,768 | 3,005 | Abnormal |
| 252875 | AS | 0,841 | -16,449 | 5,595 | 5,991 | 3,498 | Abnormal |
| 252878 | AS | 1,050 | -17,345 | 9,499 | 4,515 | 0,560 | Abnormal |
| 252879 | AS | 2,112 | -18,013 | 8,914 | 5,025 | 1,238 | Abnormal |
| 252881 | AS | 1,848 | -18,218 | 9,253 | 5,180 | 1,182 | Abnormal |
| 252883 | AS | 0,676 | -17,335 | 9,754 | 4,619 | 0,882 | Abnormal |

Table 4. Calibration data of a model based on combined TGF-β and FOXO activities using GSE10006 dataset. Each line represents an individual sample. Sample "NS" denotes a sample from a non-smoker (calibration normal samples). Sample "AS" denotes a sample of a smoker (calibration abnormal samples). All samples passed quality control.

| array (GSM...) | Sample | pathway activity (log2odds scores) | | Distance of sample to | | Detected as |
|---|---|---|---|---|---|---|
| | | FOXO | TGF-β | calibration normal | calibration abnormal | |
| 252856 | NS | 4,449 | -12,602 | 1,862 | 5,924 | Normal |
| 252857 | NS | 1,184 | -12,918 | 1,535 | 4,517 | Normal |
| 252858 | NS | 6,422 | -14,465 | 3,936 | 6,164 | Normal |
| 252859 | NS | 2,371 | -15,759 | 2,566 | 2,151 | Abnormal |
| 252860 | NS | -0,229 | -12,387 | 3,034 | 5,197 | Normal |
| 252861 | NS | 2,368 | -13,185 | 0,324 | 4,456 | Normal |
| 252862 | NS | -0,160 | -15,790 | 3,843 | 2,022 | Abnormal |
| 252863 | NS | 3,565 | -13,490 | 0,916 | 4,693 | Normal |
| 252864 | NS | 2,695 | -13,690 | 0,477 | 4,100 | Normal |
| 252865 | NS | 3,789 | -11,151 | 2,337 | 6,865 | Normal |
| 252866 | NS | 3,143 | -9,907 | 3,337 | 7,819 | Normal |
| 252871 | AS | -0,753 | -17,177 | 5,251 | 1,791 | Abnormal |
| 252874 | AS | 1,362 | -17,486 | 4,475 | 0,347 | Abnormal |
| 252875 | AS | 0,841 | -16,449 | 3,728 | 0,999 | Abnormal |
| 252878 | AS | 1,050 | -17,345 | 4,446 | 0,092 | Abnormal |
| 252879 | AS | 2,112 | -18,013 | 4,835 | 1,238 | Abnormal |
| 252881 | AS | 1,848 | -18,218 | 5,076 | 1,142 | Abnormal |
| 252883 | AS | 0,676 | -17,335 | 4,588 | 0,357 | Abnormal |

Smokers with COPD form the same dataset were used to validate the model. The results are shown further below in Tables 5 to 7.

*Linear model*

Another computational model was developed which provides a linear (small/large) airway pathway abnormality score. In this example, a score of 1 point was assigned to each abnormal pathway activity. A pathway activity was considered as abnormal if the measured TGF-β or Notch pathway or the FOXO activity was below the normal non-smoker mean-1SD or -2SD (for calculation of these thresholds, see Table 2 above and Figure 2A). Otherwise, a score of "0" was assigned to the respective pathway. The points were summed up to indicate a likelihood that the airway epithelium is abnormal and the patient is potentially at high risk for development of lung cancer. The higher the score, the more likely that the epithelium is abnormal and at risk for development of lung cancer. The score is called an APAS score (Airway Pathway Activity Score). A score of 0 is normal (low risk), a score of 3 is maximal and indicates abnormal epithelium and is assumed to confer highest risk at development of lung cancer.

**Validation of the computational models**

Subsequently for validation purposes, independent data from small airway epithelium from the GSE10006 dataset from smokers, either with early chronic obstructive pulmonary disease (COPD) or with long-standing COPD, were used to score abnormal epithelial status in smokers.

*Calculation of an airway epithelial pathway abnormality score (APAS) using a linear computational model*

In this experiment, a threshold was calculated based on mean and variance of the respective pathway activities measured in epithelial cells of small airways of healthy non-smokers ("normal" airway epithelium). More specifically, the mean and standard deviation of the TGF-$\beta$, Notch and FOXO activity in normal epithelial cells were determined by pathway analysis as described herein. The values along with resulting 1SD and 2SD thresholds are summarized in Table 2 and the passage following this table and depicted in Fig. 2A.

As shown exemplarily for TGF-$\beta$ pathway using dataset GSE10006 (cf. Figs. 2A and 2B), the threshold was defined as mean pathway activity minus 2SD. The threshold indicates transition from normal to abnormal low TGF-$\beta$ activity. The 2SD threshold is depicted for small airway epithelium. A sample was considered to have abnormal pathway activity if the pathway activity was determined to be below the defined threshold/horizontal line. It can be seen that a subgroup of smokers have abnormal TGF-$\beta$ pathway activity, in particular in the small airway (cf. Fig. 2B). In this case, abnormal pathway activity is characterized by loss of tumor suppressive TGF-$\beta$ pathway activity. Subjects from this group are assumed to be at increased risk for developing airway cancer.

For the linear model, based on these thresholds, pathway abnormality factors were assigned for each of the assessed pathway activities. A factor of "1" was assigned when the pathway activity was determined to be abnormal, otherwise "0". For example, when the activity of the TGF-$\beta$ pathway was determined to be below this threshold value, a score of 1 point was assigned to the respective pathway. Corresponding remarks apply with respect to FOXO activity. The pathway abnormality factors were then summed up to yield the APAS score.

Deviations from normal pathway activities in small airway epithelium were evaluated for validation purposes using the linear model, and the APAS score was determined based on combined TGF-$\beta$ pathway activity and FOXO transcription factor activity. An APAS score of "0" denotes normal airway epithelium (low risk). The higher the score, the higher the probability that the airway epithelium is abnormal. All samples that were used for validation passed QC as described herein. The validation results are shown in Table 5, along with calibration data.

Table 5. Calibration and validation results of a linear model based on combined TGF-$\beta$ and FOXO activities. Calibration results correspond to those shown in Table 4. Validation was performed using samples from smokers with early (samples "eCOPD") and established COPD (samples "COPD") from GSE10006 dataset. All samples passed QC as described herein.

| array (GSM...) | Sample | pathway activity (log2odds scores) | | FOXO score | TGF-$\beta$ score | APAS (N=2) |
|---|---|---|---|---|---|---|
| | | **FOXO** | **TGF-$\beta$** | | | |
| 252856 | NS | 4,449 | -12,602 | 0 | 0 | 0 |
| 252857 | NS | 1,184 | -12,918 | 0 | 0 | 0 |
| 252858 | NS | 6,422 | -14,465 | 0 | 0 | 0 |
| 252859 | NS | 2,371 | -15,759 | 0 | 1 | 1 |
| 252860 | NS | -0,229 | -12,387 | 1 | 0 | 1 |
| 252861 | NS | 2,368 | -13,185 | 0 | 0 | 0 |
| 252862 | NS | -0,160 | -15,790 | 1 | 1 | 2 |
| 252863 | NS | 3,565 | -13,490 | 0 | 0 | 0 |
| 252864 | NS | 2,695 | -13,690 | 0 | 0 | 0 |
| 252865 | NS | 3,789 | -11,151 | 0 | 0 | 0 |
| 252866 | NS | 3,143 | -9,907 | 0 | 0 | 0 |
| 252871 | AS | -0,753 | -17,177 | 1 | 1 | 2 |
| 252874 | AS | 1,362 | -17,486 | 0 | 1 | 1 |
| 252875 | AS | 0,841 | -16,449 | 0 | 1 | 1 |
| 252878 | AS | 1,050 | -17,345 | 0 | 1 | 1 |
| 252879 | AS | 2,112 | -18,013 | 0 | 1 | 1 |
| 252881 | AS | 1,848 | -18,218 | 0 | 1 | 1 |
| 252883 | AS | 0,676 | -17,335 | 0 | 1 | 1 |

(continued)

| array (GSM...) | Sample | pathway activity (log2odds scores) | | FOXO score | TGF-β score | APAS (N=2) |
|---|---|---|---|---|---|---|
| | | FOXO | TGF-β | | | |
| 252828 | COPD | 0,053 | -15,986 | 0 | 1 | 1 |
| 252829 | COPD | -3,436 | -16,539 | 1 | 1 | 2 |
| 252831 | COPD | -0,228 | -17,521 | 1 | 1 | 2 |
| 252835 | COPD | 0,843 | -16,892 | 0 | 1 | 1 |
| 252836 | COPD | 2,050 | -15,571 | 0 | 1 | 1 |
| 252837 | COPD | 1,828 | -17,016 | 0 | 1 | 1 |
| 252838 | COPD | 2,501 | -18,209 | 0 | 1 | 1 |
| 252839 | COPD | -1,155 | -18,334 | 1 | 1 | 2 |
| 252841 | COPD | -1,643 | -17,238 | 1 | 1 | 2 |
| 252844 | eCOPD | 0,889 | -15,116 | 0 | 0 | 0 |
| 252845 | eCOPD | 0,473 | -14,543 | 0 | 0 | 0 |
| 252846 | eCOPD | 0,511 | -17,705 | 0 | 1 | 1 |
| 252847 | eCOPD | 3,451 | -10,004 | 0 | 0 | 0 |
| 252848 | eCOPD | -0,713 | -18,008 | 1 | 1 | 2 |
| 252849 | eCOPD | -0,044 | -18,029 | 0 | 1 | 1 |
| 252850 | eCOPD | 2,226 | -15,501 | 0 | 1 | 1 |
| 252851 | eCOPD | 5,624 | -17,559 | 0 | 1 | 1 |
| 252854 | eCOPD | -1,087 | -17,588 | 1 | 1 | 2 |

Using the linear model the incidence of individuals with abnormal airway epithelium was highest in the smoker group with established COPD, and less in the group with early COPD. This is in line with the expected overall risk at lung cancer in COPD patients. Therefore the model performed as expected.

*Validation of the Centroid model*

Smokers with early and established COPD from the same dataset (GSE10006) were used to validate this model. This model calculates a distance score of the pathway activities found in a sample to the cluster of normal/healthy pathway activities, and to the abnormal epithelium pathway activities; based on the calibration of the model , this score defines whether the analyzed sample is considered normal small airway epithelium or abnormal. The model can be used with combined TGF-β and FOXO activities (cf. Table 6) or with combined TGF-β, FOXO and Notch activities (cf. Table 7).

Table 6. Validation data of a centroid model based on combined FOXO and TGF-β activities using samples from smokers with early and established COPD from GSE10006 dataset. All samples passed QC as described herein.

| array (GSM...) | Sample | pathway activity (log2odds scores) | | Distance of sample to... | | Detected as |
|---|---|---|---|---|---|---|
| | | FOXO | TGF-β | calibration normal | calibration abnormal | |
| 252828 | COPD | 0,053 | -15,986 | 3,828 | 1,739 | Abnormal |
| 252829 | COPD | -3,436 | -16,539 | 6,971 | 4,543 | Abnormal |
| 252831 | COPD | -0,228 | -17,521 | 5,204 | 1,250 | Abnormal |
| 252835 | COPD | 0,843 | -16,892 | 4,117 | 0,568 | Abnormal |
| 252836 | COPD | 2,050 | -15,571 | 2,444 | 2,127 | Abnormal |
| 252837 | COPD | 1,828 | -17,016 | 3,899 | 0,910 | Abnormal |

(continued)

| array (GSM...) | Sample | pathway activity (log2odds scores) | | Distance of sample to... | | Detected as |
|---|---|---|---|---|---|---|
| | | FOXO | TGF-β | calibration normal | calibration abnormal | |
| 252838 | COPD | 2,501 | -18,209 | 5,000 | 1,673 | Abnormal |
| 252839 | COPD | -1,155 | -18,334 | 6,404 | 2,354 | Abnormal |
| 252841 | COPD | -1,643 | -17,238 | 5,915 | 2,669 | Abnormal |
| 252844 | eCOPD | 0,889 | -15,116 | 2,620 | 2,320 | Abnormal |
| 252845 | eCOPD | 0,473 | -14,543 | 2,586 | 2,940 | Normal |
| 252846 | eCOPD | 0,511 | -17,705 | 4,993 | 0,577 | Abnormal |
| 252847 | eCOPD | 3,451 | -10,004 | 3,298 | 7,816 | Normal |
| 252848 | eCOPD | -0,713 | -18,008 | 5,880 | 1,825 | Abnormal |
| 252849 | eCOPD | -0,044 | -18,029 | 5,538 | 1,219 | Abnormal |
| 252850 | eCOPD | 2,226 | -15,501 | 2,335 | 2,277 | Abnormal |
| 252851 | eCOPD | 5,624 | -17,559 | 5,243 | 4,607 | Abnormal |
| 252854 | eCOPD | -1,087 | -17,588 | 5,780 | 2,112 | Abnormal |

Table 7. Validation data of a centroid model based on combined TGF-β, NOTCH and FOXO activities using samples from smokers with early and established COPD from GSE10006 dataset. All samples passed QC as described herein.

| array (GSM...) | Sample | pathway activity (log2odds scores) | | | Distance of sample to... | | Detected as |
|---|---|---|---|---|---|---|---|
| | | FOXO | TGF-β | NOTCH | calibration normal | calibration abnormal | |
| 252828 | COPD | 0,053 | -15,986 | 11,070 | 3,907 | 2,744 | Abnormal |
| 252829 | COPD | -3,436 | -16,539 | 8,373 | 7,229 | 4,580 | Abnormal |
| 252831 | COPD | -0,228 | -17,521 | 6,956 | 6,177 | 2,351 | Abnormal |
| 252835 | COPD | 0,843 | -16,892 | 10,237 | 4,117 | 1,409 | Abnormal |
| 252836 | COPD | 2,050 | -15,571 | 10,095 | 2,451 | 2,417 | Abnormal |
| 252837 | COPD | 1,828 | -17,016 | 9,870 | 3,921 | 1,296 | Abnormal |
| 252838 | COPD | 2,501 | -18,209 | 7,639 | 5,657 | 2,124 | Abnormal |
| 252839 | COPD | -1,155 | -18,334 | 9,498 | 6,452 | 2,417 | Abnormal |
| 252841 | COPD | -1,643 | -17,238 | 11,369 | 6,013 | 3,604 | Abnormal |
| 252844 | eCOPD | 0,889 | -15,116 | 11,327 | 2,820 | 3,324 | Normal |
| 252845 | eCOPD | 0,473 | -14,543 | 12,743 | 3,567 | 4,801 | Normal |
| 252846 | eCOPD | 0,511 | -17,705 | 7,115 | 5,915 | 1,921 | Abnormal |
| 252847 | eCOPD | 3,451 | -10,004 | 6,562 | 4,974 | 8,172 | Normal |
| 252848 | eCOPD | -0,713 | -18,008 | 8,389 | 6,178 | 1,909 | Abnormal |
| 252849 | eCOPD | -0,044 | -18,029 | 10,181 | 5,539 | 1,735 | Abnormal |
| 252850 | eCOPD | 2,226 | -15,501 | 9,984 | 2,355 | 2,502 | Normal |
| 252851 | eCOPD | 5,624 | -17,559 | 7,676 | 5,857 | 4,779 | Abnormal |
| 252854 | eCOPD | -1,087 | -17,588 | 9,134 | 5,894 | 2,120 | Abnormal |

It can be seen that the model scores some early COPD samples as normal, but always scores abnormal for the established/longstanding COPD samples, which is in line with the expected risk at lung cancer in these two groups.

## Clinical use of the method

Clearly not all (heavy) smokers will develop lung cancer, reason why the airway abnormality factor and/or risk score is only detected in a subgroup of the smokers. This variation among smokers also indicates the need for the means and methods of the present invention, which allows identification of subjects, in particular smokers, with abnormal airway epithelium.

The present invention is expected to reduce unnecessary invasive diagnostic procedures in heavy smokers, and may enable early minimally invasive treatment of high risk patients.

In addition, pathway analysis on airway epithelial cells may be an additional diagnostic test for lung cancer, enabling choice of systemic (targeted) therapy.

1. Smokers can be screened for premalignant changes with high risk for development of lung cancer, using the described method and avoiding repeated exposure to radiation associated with imaging modalities like CT scans for screening. Epithelial cells can be obtained from the airway epithelium, and determination of combined TGF-$\beta$/Notch/ PI3K-FOXO pathway activity or at least one of these pathway activities, preferably Notch and TGF-$\beta$, can be performed. Results can be interpreted in the screening model to predict risk of presence of a premalignant change, indicating high risk at development of smoking-associated lung cancer.

2. If a smoker presents with a complaint (e.g. a cough or shortness of breath) or an abnormal imaging finding, using e.g. a BAL epithelial cells can be obtained from the airway epithelium, and determination of combined TGF-$\beta$/Notch/ PI3K-FOXO pathway activity can be performed, and results interpreted in the screening model to predict risk for presence of a premalignant change, indicating high risk for development of smoking-associated lung cancer.

3. If a lesion is detected in the lungs on an image like chest radiograph or CT, and it is not known whether this is a malignant lesion, performing the pathway analysis on BAL cells provides complementary information on the character of the lesion, as to it potential malignancy, especially for smokers.

4. A classifier based on pathway activity can be integrated in the read-out of diagnostic procedures, e.g. bronchoscopy, to improve the sensitivity/specificity of the technique to assess the probability of lung cancer.

5. In any of the above scenarios, the calculated risk scores can be used to stratify patients for close monitoring and/or chemoprevention.

6. Smokers can be screened for pre-malignant changers with high risk at development of lung cancer, using the described method and avoiding repeated exposure to radiation associated with imaging modalities like CT scans for screening. Using a BAL or other means epithelial cells can be obtained from the airway epithelium, and determination of combined TGF-$\beta$/ PI3K-FOXO pathway activity can be performed, and results interpreted in the computational model to provide an airway epithelium abnormality score, indicating the probability of a pre-malignant change being present, which indicates risk at development of (squamous) lung cancer.

7. If a smoker presents with a complaint, like a persistent cough, using a BAL or trachea brushing technology epithelial cells can be obtained from the airway epithelium, and on RNA isolated from this cell sample, defined mRNA measurement are performed for determination of combined TGF-$\beta$/HH/PI3K-FOXO pathway activity, and results interpreted in the computational model to provide an airway epithelium abnormality score, indicating the probability of a pre-malignant change being present, which indicates risk at development of (squamous) lung cancer.

8. If a lesion is detected in the lungs on an image like chest radiograph or CT, and it is not known whether this is a malignant lesion, performing the described test on BAL/brushing-obtained airway epithelial cells provides complementary information on the character of the lesion, as to its potential malignancy, especially also for smokers.

## CDS application

With reference to Fig. 3 (diagrammatically showing a clinical decision support (CDS) system configured to determine a risk score that indicates a risk that a subject will develop an airway cancer, as disclosed herein), a clinical decision support (CDS) system 10 is implemented as a suitably configured computer 12. The computer 12 may be configured to operate as the CDS system 10 by executing suitable software, firmware, or other instructions stored on a non-transitory storage medium (not shown), such as a hard drive or other magnetic storage medium, an optical disk or another optical storage medium, a random access memory (RAM), a read-only memory (ROM), a flash memory, or another electronic storage medium, a network server, or so forth. While the illustrative CDS system 10 is embodied by the illustrative computer 12, more generally the CDS system may be embodied by a digital processing device or an apparatus comprising a digital processor configured to perform clinical decision support methods as set forth herein. For example, the digital processing device may be a handheld device (e.g., a personal data assistant or smartphone running a CDS application), a notebook computer, a desktop computer, a tablet computer or device, a remote network server, or so forth. The computer 12 or other digital processing device typically includes or is operatively connected with a display device 14 via which information including clinical decision support recommendations are displayed to medical personnel. The computer 12 or other digital processing device typically also includes or is operatively connected with one or more user input devices, such as an illustrative keyboard 16, or a mouse, a trackball, a trackpad, a touch-sensitive screen (possibly integrated with the display device 14), or another pointer-based user input device, via which medical personnel can input information such as operational commands for controlling the CDS system 10, data for use by the CDS system 10, or so forth.

The CDS system 10 receives as input information pertaining to a subject (e.g., a hospital patient, or an outpatient being treated by an oncologist, physician, or other medical personnel, or a person undergoing cancer screening or some other medical diagnosis who is known or suspected to have a certain type of airway cancer, or a predisposition for developing an airway cancer. The CDS system 10 applies various data analysis algorithms to this input information in order to generate clinical decision support recommendations that are presented to medical personnel via the display device 14 (or via a voice synthesizer or other device providing human-perceptible output). In some embodiments, these algorithms may include applying a clinical guideline to the patient. A clinical guideline is a stored set of standard or "canonical" treatment recommendations, typically constructed based on recommendations of a panel of medical experts and optionally formatted in the form of a clinical "flowchart" to facilitate navigating through the clinical guideline. In various embodiments the data processing algorithms of the CDS 10 may additionally or alternatively include various diagnostic or clinical test algorithms that are performed on input information to extract clinical decision recommendations, such as machine learning methods disclosed herein.

In the illustrative CDS systems disclosed herein (e.g., CDS system 10), the CDS data analysis algorithms include one or more diagnostic or clinical test algorithms that are performed on input genomic and/or proteomic information acquired by one or more medical laboratories 18. These laboratories may be variously located "on-site", that is, at the hospital or other location where the subject is undergoing medical examination and/or treatment, or "off-site", e.g., a specialized and centralized laboratory that receives (via mail or another delivery service) a sample of the subject that has been extracted from the subject.

The sample is processed by the laboratory to generate genomic or proteomic information. For example, the sample may be processed using a microarray (also variously referred to in the art as a gene chip, DNA chip, biochip, or so forth) or by quantitative polymerase chain reaction (qPCR) processing to measure probative genomic or proteomic information such as expression levels of genes of interest, for example in the form of a level of messenger ribonucleic acid (mRNA) that is transcribed from the gene, or a level of a protein that is translated from the mRNA transcribed from the gene. As another example, the sample may be processed by a gene sequencing laboratory to generate sequences for deoxyribonucleic acid (DNA), or to generate an RNA sequence, copy number variation, methylation, or so forth. Other contemplated measurement approaches include immunohistochemistry (IHC), cytology, fluorescence in situ hybridization (FISH), proximity ligation assay or so forth, performed on a pathology slide. Other information that can be generated by microarray processing, mass spectrometry, gene sequencing, or other laboratory techniques includes methylation information. Various combinations of such genomic and/or proteomic measurements may also be performed.

In some embodiments, the medical laboratories 18 perform a number of standardized data acquisitions on the sample of the subject, so as to generate a large quantity of genomic and/or proteomic data. For example, the standardized data acquisition techniques may generate an (optionally aligned) DNA sequence for one or more chromosomes or chromosome portions, or for the entire genome. Applying a standard microarray can generate thousands or tens of thousands of data items such as expression levels for a large number of genes, various methylation data, and so forth. Similarly, PCR-based measurements can be used to measure the expression level of a selection of genes. This plethora of genomic and/or proteomic data, or selected portions thereof, are input to the CDS system 10 to be processed so as to develop clinically useful information for formulating clinical decision support recommendations.

The disclosed CDS systems and related methods relate to processing of genomic and/or proteomic data to assess activity of various cellular signaling pathways and to determine a risk score that indicates a risk that a subject will develop an airway cancer. However, it is to be understood that the disclosed CDS systems (e.g., CDS system 10) may optionally further include diverse additional capabilities, such as generating clinical decision support recommendations in accordance with stored clinical guidelines based on various patient data such as vital sign monitoring data, patient history data, patient demographic data (e.g., gender, age, or so forth), patient medical imaging data, or so forth. Alternatively, in some embodiments the capabilities of the CDS system 10 may be limited to only performing genomic and/or proteomic data analyses to assess the activity of cellular signaling pathways and to determine a risk score that indicates whether a subject has abnormal airway epithelium and/or is at risk of developing an airway cancer, as disclosed herein.

With continuing reference to exemplary Fig. 3, the CDS system 10 infers activity 22 of two or more cellular signaling pathways selected from the group consisting of a TGF-$\beta$ pathway, a PI3K-FOXO pathway and a Notch pathway (Pt, $P_p$, $P_n$), in the subject based on, but not restricted to, the expression levels 20 of one or more target gene(s) of the cellular signaling pathways measured in the sample of the subject.

Measurement of mRNA expression levels of genes that encode for regulatory proteins of the cellular signaling pathway, such as an intermediate protein that is part of a protein cascade forming the cellular signaling pathway, is an indirect measure of the regulatory protein expression level and may or may not correlate strongly with the actual regulatory protein expression level (much less with the overall activity of the cellular signaling pathway). The cellular signaling pathway directly regulates the transcription of the target genes - hence, the expression levels of mRNA transcribed from the target genes is a direct result of this regulatory activity. Hence, the CDS system 10 infers activity of the two or more cellular signaling pathways based on expression levels of one or more target gene(s) (mRNA or protein level as a surrogate measurement) of the cellular signaling pathways. This ensures that the CDS system 10 infers the activity of the pathway based on direct information provided by the measured expression levels of the target gene(s).

The inferred activities are then used to determine 24 a risk score that indicates a risk that the subject will develop an airway cancer, as described in detail herein. The risk score is based on a combination of the inferred activities. For example, the risk score may be the "Multi-Pathway Score" (*MPS*) calculated as described in detail herein and in the following reference: WO2014174003, WO2016062892 and WO2016062893.

Based on the determined *MPS,* the CDS system 10, in this example, assigns 26 the subject to at least one of a plurality of risk groups associated with different indicated risks that the subject will develop an airway cancer, and/or decides 28 a treatment recommended for the subject based on the indicated risk.

It is further possible that the CDS system 10 is configured to combine the risk score with one or more additional risk scores obtained from one or more additional prognostic tests to obtain a combined risk score, wherein the combined risk score indicates a risk that the subject will develop an airway cancer.

## Claims

1. A computer-implemented method for determining whether a subject has abnormal airway epithelium, performed by a digital processing device, wherein the determining comprises:

   determining an airway abnormality factor indicating whether the subject has abnormal airway epithelium based on a combination of activities of cellular signaling pathways in an epithelial cell sample derived from an airway of the subject,
   wherein the cellular signaling pathways comprise two or more cellular signaling pathways selected from the group consisting of a TGF-β pathway, a PI3K-FOXO pathway, and a Notch pathway,
   wherein the determining of the signaling pathway abnormality factor is further based on a reference activity of the respective cellular signaling pathway,
   wherein the reference activity reflects activity of the respective cellular signaling pathway found in airway epithelium of healthy subjects,
   wherein the determining of the airway abnormality factor comprises:

   determining a signaling pathway abnormality factor for each of the respective cellular signaling pathways based on the activity of the respective cellular signaling pathway in the epithelial cell sample and determining the airway abnormality factor based on a combination of the determined cellular signaling pathway abnormality factors,
   wherein a quantitative measurement of signal transduction pathway activity in epithelial cells obtained from upper or lower airways is based on inferring activity of a signal transduction pathway from measurements of mRNA levels of three or more validated direct target genes of the transcription factor associated with the respective signaling pathway.

2. A computer-implemented method for determining a risk score that indicates a risk that a subject having abnormal airway epithelium will develop an airway cancer performed by a digital processing device, wherein the determining comprises:

   determining the risk score based on a combination of the activities of cellular signaling pathways in an epithelial cell sample derived from an airway of the subject,
   wherein the cellular signaling pathways comprise two or more cellular signaling pathways selected from the group consisting of a TGF-β pathway, a PI3K-FOXO pathway, and a Notch pathway,
   wherein the determining of the risk score is further based on a combination of reference activities of the cellular signaling pathways, and
   wherein the risk score is defined such that the indicated risk increases with a decreasing activity of the TGF-β pathway and one or more of an increasing activity of the PI3K pathway, and/or a decreasing activity of the Notch pathway with respect to the reference activities of the cellular signaling pathways,
wherein the determining of the risk score comprises:

determining an airway abnormality factor based on the combination of the activities of the cellular signaling pathways in the epithelial cell sample and translating the airway abnormality factor into the risk score, wherein the determining of the airway abnormality factor comprises:

determining a signaling pathway abnormality factor for each of the respective cellular signaling pathways based on the activity of the respective cellular signaling pathway in the epithelial cell sample and determining the airway abnormality factor based on a combination of the determined cellular signaling pathway abnormality factors,
wherein a quantitative measurement of signal transduction pathway activity in epithelial cells obtained from upper or lower airways is based on inferring activity of a signal transduction pathway from measurements of mRNA levels of three or more validated direct target genes of the transcription factor associated with the respective signaling pathway.

3. The method according to any of the preceding claims,
wherein the airway abnormality factor and the risk score, respectively, is determined based on evaluating a calibrated mathematical model relating the activities of the cellular signaling pathways in the epithelial cell sample to the airway abnormality factor and the risk score, respectively,

4. The method according to claim 3, wherein the calibrated mathematical pathway model is a probabilistic model, preferably a Bayesian network model, based on conditional probabilities relating the activity level of the signaling pathway associated TF element and the expression levels of the three or more target genes, or the calibrated mathematical pathway model may be based on one or more linear combination(s) of the expression levels of the three or more target genes.

5. The method according to any of the preceding claims,
wherein the activities of the cellular signaling pathways in the epithelial cell sample is inferred or inferable by a method comprising:

receiving expression levels of one and preferably three or more target genes of each of the respective cellular signaling pathway,
determining an activity level of a cellular signaling pathway associated transcription factor (TF) element, the cellular signaling pathway associated TF element controlling transcription of the one and preferably three or more target genes, the determining being based on evaluating a calibrated mathematical pathway model relating expression levels of the target gene(s) to the activity level of the respective cellular signaling pathway, and

- inferring the activity of the respective cellular signaling pathway based on the determined activity level of the cellular signaling pathway associated TF element.

6. The method of any of the preceding claims,

wherein the cellular signaling pathways comprise the TGF-β pathway and one or more of the PI3K-FOXO pathway, and the Notch pathway,
preferably the PI3K-FOXO pathway and at least the PI3K-FOXO pathway.

7. The method of any of the preceding claims, wherein:

the three or more TGF-β target genes are selected from the group consisting of: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKN1A, CTGF, GADD45B, ID1, IL11, JUNB, SERPINE1, PDGFB, SKIL, SMAD7, SNAI2, and VEGFA, most preferably, from the group consisting of: ANGPTL4, CDC42EP3, ID1, IL11, JUNB, SERPINE1, SKIL, and SMAD7, or wherein the three or more TGF-β target genes are selected from the group consisting of: CDC42EP3, GADD45B, HMGA2, ID1, JUNB, OVAL1, VEGFA, SGK1,
and/or
the three or more PI3K-FOXO target genes are selected from the group consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2, TNFSF10, preferably, from the group consisting of: FBXO32, BCL2L11, SOD2, TNFSF10, BCL6, BTG1, CCNG2, CDKN1B, BNIP3, GADD45A, INSR, and MXI1,

and/or

the three or more Notch target genes are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target gene(s) are selected from the group consisting of: DTX1, HES1, HES4, HES5, HEY2, MYC, NRARP, and PTCRA, and one or more Notch target gene(s) are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC.

8. The method of any of the preceding claims,
wherein the method further comprises:

providing additional evidence for a non-diagnostic nodule being malignant or benign, and/or
prediction whether airway epithelium is pre-malignant, and/or
prediction whether a person has a high risk at development of airway cancer, and/or
prediction whether a person has a high risk at development of lung cancer, and/or prediction whether a person has a high risk at development of squamous lung cancer, and/or
prediction whether a person has a high risk at development of lung adenocarcinoma, and/or
prediction whether a person can benefit from a local therapy to prevent development of cancer, and/or
prediction whether a patient has lung cancer, and/or
prognosis and/or prediction, and/or
prediction of drug efficacy of e.g. chemotherapy and/or hormonal treatment, and/or
monitoring of drug efficacy, and/or
deciding on a frequency of monitoring or, more particularly, on a frequency of therapy response monitoring, and/or
drug development, and/or
assay development, and/or
prediction whether a person is at risk of developing invasive airway cancer, and/or
prediction whether a person is at risk of disease progression, and/or
prediction or diagnosis whether a person has reduced risk after treatment (e.g. chemoprevention), and/or
complementing diagnostic information coming from other modalities (e.g. imaging) and/or other pathological and/or genetic testing, and/or
cancer staging.

9. An apparatus for determining an airway abnormality factor indicating whether a subject has abnormal airway epithelium or a risk score that indicates a risk that a subject having abnormal airway epithelium will develop an airway cancer comprising a digital processor configured to perform the method of any of claims 1 to 8.

10. A non transitory storage medium for determining an airway abnormality factor indicating whether a subject has abnormal airway epithelium or a risk score that indicates a risk that a subject having abnormal airway epithelium will develop an airway cancer storing instructions that are executable by a digital processing device to perform the method of any of claims 1 to 8.

11. A computer program for determining an airway abnormality factor indicating whether a subject has abnormal airway epithelium or a risk score that indicates a risk that a subject having abnormal airway epithelium will develop an airway cancer comprising program code means for causing a digital processing device to perform a method of any of claims 1 to 8, when the computer program is run on the digital processing device.

12. A kit for determining an airway abnormality factor indicating whether a subject has abnormal airway epithelium or a risk score that indicates a risk that a subject having abnormal airway epithelium will develop an airway cancer, the kit comprising:

components for determining the expression levels of at least three target genes of a TGF-β cellular signaling pathway, at least three target genes of a PI3K-FOXO cellular signaling pathway, at least three target genes of a Notch cellular signaling pathway, and
the apparatus of claim 9, the non-transitory storage medium of claim 10, or the computer program of claim 11.

13. A method for *in vitro* or *ex vivo* diagnosing or prognosticating whether a subject has abnormal airway epithelium or whether a subject having abnormal airway epithelium will develop an airway cancer using a kit, the kit comprising components for determining the expression levels of at least three target genes of a TGF-β cellular signaling pathway, at least three target genes of a PI3K-FOXO cellular signaling pathway, at least three target genes of a Notch cellular signaling pathway,

the method comprising providing an extracted epithelial cell sample from an airway of the subject, and subjecting the extracted epithelial cell sample to the method according to any one of claims 1 to 7.

14. A method for *in vitro* or *ex vivo* diagnosing or prognosticating whether a subject has abnormal airway epithelium or whether a subject having abnormal airway epithelium will develop an airway cancer using a kit, the kit comprising components for determining the expression levels of at least three target genes of a TGF-β cellular signaling pathway, at least three target genes of a PI3K-FOXO cellular signaling pathway, at least three target genes of a Notch cellular signaling pathway, the method comprising:

determining an airway abnormality factor indicating whether the subject has abnormal airway epithelium based on a combination of activities of cellular signaling pathways in an epithelial cell sample derived from an airway of the subject,

wherein the cellular signaling pathways comprise two or more cellular signaling pathways selected from the group consisting of a TGF-β pathway, a PI3K-FOXO pathway, and a Notch pathway,

wherein the determining of the signaling pathway abnormality factor is further based on a reference activity of the respective cellular signaling pathway,

wherein the reference activity reflects activity of the respective cellular signaling pathway found in airway epithelium of healthy subjects, and

wherein the determining of the airway abnormality factor comprises:

determining a signaling pathway abnormality factor for each of the respective cellular signaling pathways based on the activity of the respective cellular signaling pathway in the epithelial cell sample and determining the airway abnormality factor based on a combination of the determined cellular signaling pathway abnormality factors,

wherein a quantitative measurement of signal transduction pathway activity in epithelial cells obtained from upper or lower airways is based on inferring activity of a signal transduction pathway from measurements of mRNA levels of three or more validated direct target genes of the transcription factor associated with the respective signaling pathway.

15. The method for *in vitro* or *ex vivo* diagnosing or prognosticating whether a subject has abnormal airway epithelium or whether a subject having abnormal airway epithelium will develop an airway cancer according to claim 13 or 14, wherein

the three or more TGF-β target genes are selected from the group consisting of: ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKN1A, CTGF, GADD45B, ID1, IL11, JUNB, SERPINE1, PDGFB, SKIL, SMAD7, SNAI2, and VEGFA, most preferably, from the group consisting of: ANGPTL4, CDC42EP3, ID1, IL11, JUNB, SERPINE1, SKIL, and SMAD7, or wherein the three or more TGF-β target genes are selected from the group consisting of: CDC42EP3, GADD45B, HMGA2, ID1, JUNB, OVAL1, VEGFA, SGK1,

and/or

the three or more PI3K-FOXO target genes are selected from the group consisting of: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2, TNFSF10, preferably, from the group consisting of: FBXO32, BCL2L11, SOD2, TNFSF10, BCL6, BTG1, CCNG2, CDKN1B, BNIP3, GADD45A, INSR, and MXI1,

and/or

the three or more Notch target genes are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target gene(s) are selected from the group consisting of: DTX1, HES1, HES4, HES5, HEY2, MYC, NRARP, and PTCRA, and one or more Notch target gene(s) are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC.

**Patentansprüche**

1. Computergestütztes Verfahren zum Bestimmen, ob ein Proband ein anomales Atemwegsepithel aufweist, durchgeführt von einer digitalen Verarbeitungsvorrichtung, wobei das Bestimmen Folgendes umfasst:

   Bestimmen eines Atemweg-Anomaliefaktors, der angibt, ob der Proband ein anomales Atemwegsepithel aufweist, basierend auf einer Kombination von Aktivitäten zellulärer Signalwege in einer aus den Atemwegen des Probanden gewonnenen Epithelzellprobe,
   wobei die zellulären Signalwege zwei oder mehr zelluläre Signalwege umfassen, die aus der Gruppe bestehend aus einem TGF-β-Signalweg, einem PI3K-FOXO-Signalweg und einem Notch-Signalweg ausgewählt sind,
   wobei das Bestimmen des Signalweg-Anomaliefaktors weiter auf einer Referenzaktivität des jeweiligen zellulären Signalwegs basiert,
   wobei die Referenzaktivität die Aktivität des jeweiligen zellulären Signalwegs im Atemwegsepithel gesunder Probanden widerspiegelt,
   wobei das Bestimmen des Atemweg-Anomaliefaktors Folgendes umfasst:
   Bestimmen eines Signalweg-Anomaliefaktors für jeden der jeweiligen zellulären Signalwege auf Basis der Aktivität des jeweiligen zellulären Signalwegs in der Epithelzellprobe und Bestimmen des Atemweg-Anomaliefaktors auf Basis einer Kombination der bestimmten zellulären Signalweg-Anomaliefaktoren,
   wobei eine quantitative Messung der Aktivität von Signaltransduktionswegen in Epithelzellen, die aus den oberen oder unteren Atemwegen erhalten wurden, auf dem Ableiten einer Aktivität eines Signaltransduktionswegs aus Messungen der mRNA-Spiegel von drei oder mehr validierten direkten Zielgenen des mit dem jeweiligen Signalweg assoziierten Transkriptionsfaktors basiert.

2. Computergestütztes Verfahren zum Bestimmen einer Risikobewertung, die das Risiko angibt, dass ein Proband, der ein anomales Atemwegsepithel aufweist, an Atemwegskrebs erkranken wird, durchgeführt von einer digitalen Verarbeitungsvorrichtung, wobei das Bestimmen Folgendes umfasst:

   Bestimmen der Risikobewertung auf Basis einer Kombination der Aktivitäten zellulärer Signalwege in einer aus den Atemwegen des Probanden gewonnenen Epithelzellprobe,
   wobei die zellulären Signalwege zwei oder mehr zelluläre Signalwege umfassen, die aus der Gruppe bestehend aus einem TGF-β-Signalweg, einem PI3K-FOXO-Signalweg und einem Notch-Signalweg ausgewählt sind,
   wobei das Bestimmen der Risikobewertung weiter auf einer Kombination von Referenzaktivitäten der zellulären Signalwege basiert, und
   wobei die Risikobewertung so definiert ist, dass das angegebene Risiko mit einer abnehmenden Aktivität des TGF-β-Signalwegs und eine oder mehrere von einer zunehmenden Aktivität des PI3K-Signalwegs und/oder einer abnehmenden Aktivität des Notch-Signalwegs im Vergleich zu den Referenzaktivitäten der zellulären Signalwege steigt,
   wobei das Bestimmen der Risikobewertung Folgendes umfasst:
   Bestimmen eines Atemweg-Anomaliefaktors auf Basis der Kombination der Aktivitäten der zellulären Signalwege in der Epithelzellprobe und Umrechnen des Atemweg-Anomaliefaktors in eine Risikobewertung,
   wobei das Bestimmen des Atemweg-Anomaliefaktors Folgendes umfasst:
   Bestimmen eines Signalweg-Anomaliefaktors für jeden der jeweiligen zellulären Signalwege auf Basis der Aktivität des jeweiligen zellulären Signalwegs in der Epithelzellprobe und Bestimmen des Atemweg-Anomaliefaktors auf Basis einer Kombination der bestimmten zellulären Signalweg-Anomaliefaktoren,
   wobei eine quantitative Messung der Aktivität von Signaltransduktionswegen in Epithelzellen, die aus den oberen oder unteren Atemwegen erhalten wurden, auf dem Ableiten einer Aktivitätt eines Signaltransduktionswegs aus Messungen der mRNA-Spiegel von drei oder mehr validierten direkten Zielgenen des mit dem jeweiligen Signalweg assoziierten Transkriptionsfaktors basiert.

3. Verfahren nach einem der vorstehenden Ansprüche
   wobei der Atemweg-Anomaliefaktor und die Risikobewertung jeweils auf Basis der Auswertung eines kalibrierten mathematischen Modells bestimmt wird, das die Aktivitäten der zellulären Signalwege in der Epithelzellprobe mit dem Atemweg-Anomaliefaktor bzw. der Risikobewertung in Beziehung setzt.

4. Verfahren nach Anspruch 3, wobei das kalibrierte mathematische Pfadmodell ein probabilistisches Modell, bevorzugt ein Bayes'sches Netzwerkmodell, ist, das auf bedingten Wahrscheinlichkeiten basiert, die den Aktivitätsgrad des mit dem Signalweg assoziierten TF-Elements und die Expressionsniveaus der drei oder mehr Zielgene in Beziehung setzen, oder wobei das kalibrierte mathematische Pfadmodell auf einer oder mehreren linearen Kombinationen der Expressionsniveaus der drei oder mehr Zielgene basieren kann.

5. Verfahren nach einem der vorstehenden Ansprüche
wobei die Aktivitäten der zellulären Signalwege in der Epithelzellprobe durch ein Verfahren erschlossen werden oder erschlossen werden können, das Folgendes umfasst:

Empfangen von Expressionsniveaus von einem und bevorzugt drei oder mehr Zielgenen jedes der jeweiligen zellulären Signalwege,

Bestimmen des Aktivitätsniveaus eines mit einem zellulären Signalweg assoziierten Transkriptionsfaktor- (TF-) Elements, wobei das mit dem zellulären Signalweg assoziierte TF-Element die Transkription des einen oder bevorzugt der drei oder mehr Zielgene steuert; wobei das Bestimmen auf der Auswertung eines kalibrierten mathematischen Signalwegmodells basiert, das die Expressionsniveaus des/der Zielgene(s) mit dem Aktivitätsniveau des jeweiligen zellulären Signalwegs in Beziehung setzt;

- Erschließen der Aktivität des jeweiligen zellulären Signalwegs auf Basis des bestimmten Aktivitätsniveaus des mit dem zellulären Signalweg assoziierten TF-Elements.

6. Verfahren nach einem der vorstehenden Ansprüche,

wobei die zellulären Signalwege den TGF-β-Signalweg und einen oder mehrere des PI3K-FOXO-Signalwegs und den Notch-Signalwegs umfassen,
bevorzugt den PI3K-FOXO-Signalweg und mindestens den PI3K-FOXO-Signalweg.

7. Verfahren nach einem der vorstehenden Ansprüche, wobei:
die drei oder mehr TGF-β-Zielgeneaus der Gruppe ausgewählt sind, bestehend aus:

ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, bevorzugter aus der Gruppe bestehend aus: ANGPTL4, CDC42EP3, CDKN1A, CTGF, GADD45B, ID1, IL11, JUNE, SERPINE1, PDGFB, SKIL, SMAD7, SNAI2 und VEGFA, besonders bevorzugt aus der Gruppe bestehend aus: ANGPTL4, CDC42EP3, ID1, IL11, JUNB, SERPINE1, SKIL und SMAD7, oder wobei die drei oder mehr TGF-β-Zielgene aus der Gruppe ausgewählt sind, bestehend aus: CDC42EP3, GADD45B, HMGA2, ID1, JUNB, OVAL1, VEGFA, SGK1,
und/oder
die drei oder mehr PI3K-FOXO-Zielgene aus der Gruppe ausgewählt sind, bestehend aus: AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2, TNFSF10, bevorzugt aus der Gruppe bestehend aus: FBXO32, BCL2L11, SOD2, TNFSF10, BCL6, BTG1, CCNG2, CDKN1B, BNIP3, GADD45A, INSR und MXI1.
und/oder
die drei oder mehr Notch-Zielgene aus der Gruppe ausgewählt sind, bestehend aus: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PINI, PLXND1, PTCRA, SOX9 und TNC; wobei bevorzugt zwei oder mehr Notch-Zielgene aus der Gruppe ausgewählt sind. bestehend aus; DTX1, HES1, HES4, HES5, HEY2, MYC, NRARP und PTCRA, und ein oder mehrere Notch-Zielgene aus der Gruppe ausgewählt sind, bestehend aus: CD28, CD44, DLGAP5, EPHB3, FABP7, GFAP, GIMAP5, HES7, HEY1, HEYL, KLF5, NFKB2, NOX1, PBX1, PIN1, PLXND1, SOX9 und TNC.

8. Verfahren nach einem der vorstehenden Ansprüche,
wobei das Verfahren weiter Folgendes umfasst:

Bereitstellung zusätzlicher Anhaltspunkte darauf, ob ein nicht-diagnostischer Knoten bösartig oder gutartig ist, und/oder
Vorhersage, ob das Atemwegsepithel präkanzerös ist, und/oder
Vorhersage, ob eine Person ein hohes Risiko für die Entwicklung von Atemwegskrebs aufweist, und/oder
Vorhersage, ob eine Person ein hohes Risiko für die Entwicklung von Lungenkrebs aufweist, und/oder
Vorhersage, ob eine Person ein hohes Risiko für die Entwicklung von Plattenepithelkarzinomen der Lunge aufweist, und/oder

Vorhersage, ob eine Person ein hohes Risiko für die Entwicklung eines Lungenadenokarzinoms aufweist, und/oder

Vorhersage, ob eine Person von einer lokalen Therapie zur Vorbeugung von Krebs profitieren kann, und/oder

Vorhersage, ob ein Patient an Lungenkrebs erkrankt ist, und/oder

Prognose und/oder Vorhersage, und/oder

Vorhersage der Wirksamkeit von Arzneimitteln z. B. der Chemotherapie- und/oder Hormonbehandlung, und/oder

Überwachung der Wirksamkeit von Arzneimitteln und/oder

Festlegen der Häufigkeit der Überwachung oder insbesondere, der Häufigkeit der Überwachung des Therapieansprechens, und/oder

Arzneimittelentwicklung und/oder

Assayentwicklung, und/oder

Vorhersage, ob eine Person ein Risiko für die Entwicklung eines invasiven Atemwegskrebses aufweist, und/oder

Vorhersage, ob bei einer Person ein Risiko für das Fortschreiten der Krankheit besteht, und/oder

Vorhersage oder Diagnose, ob eine Person nach einer Behandlung (z. B. Chemoprävention) ein reduziertes Risiko aufweist, und/oder

ergänzende diagnostische Informationen aus anderen Modalitäten (z. B. Bildgebung) und/oder anderen pathologischen und/oder Gentests, und/oder

Krebsstadienbestimmung.

9. Einrichtung zum Bestimmen eines Atemweg-Anomaliefaktors, der angibt, ob ein Proband ein anomales Atemwegsepithel aufweist, oder einer Risikobewertung, die das Risiko angibt, dass ein Proband, der ein anomales Atemwegsepithel aufweist, an Atemwegskrebs erkranken wird, umfassend einen digitalen Prozessor, der so konfiguriert ist, dass er das Verfahren nach einem der Ansprüche 1 bis 8 durchführt.

10. Nichtflüchtiges Speichermedium zum Bestimmen eines Atemweg-Anomaliefaktors, der angibt, ob ein Proband ein anomales Atemwegsepithel aufweist, oder einer Risikobewertung, die das Risiko angibt, dass ein Proband, der anomales Atemwegsepithel aufweist, an Atemwegskrebs erkranken wird, das Anweisungen speichert, die von einer digitalen Verarbeitungsvorrichtung ausgeführt werden können, um das Verfahren nach einem der Ansprüche 1 bis 8 durchzuführen.

11. Computerprogramm zum Bestimmen eines Atemweg-Anomaliefaktors, der angibt, ob ein Proband ein anomales Atemwegsepithel aufweist, oder einer Risikobewertung, die das Risiko angibt, dass ein Proband, der ein anomales Atemwegsepithel aufweist, an Atemwegskrebs erkranken wird; umfassend Programmcodemittel, die eine digitales Verarbeitungsvorrichtung veranlassen, ein Verfahren nach einem der Ansprüche 1 bis 8 durchzuführen, wenn das Computerprogramm auf der digitalen Verarbeitungsvorrichtung ausgeführt wird.

12. Kit zum Bestimmen eines Atemweg-Anomaliefaktors, der angibt, ob ein Proband ein anomales Atemwegsepithel aufweist, oder einer Risikobewertung, die das Risiko angibt, dass ein Proband, der ein anomales Atemwegsepithel aufweist, an Atemwegskrebs erkranken wird; wobei das Kit Folgendes umfasst:

   Komponenten zur Bestimmung der Expressionsniveaus von mindestens drei Zielgenen eines TGF-$\beta$-Zellsignalwegs, mindestens drei Zielgenen eines PI3K-FOXO-Zellsignalwegs, mindestens drei Zielgenen eines Notch-Zellsignalwegs und

   die Einrichtung nach Anspruch 9, das nichtflüchtige Speichermedium nach Anspruch 10 oder das Computerprogramm nach Anspruch 11.

13. Verfahren zur *In-vitro*- oder Ex-vivo-Diagnostik oder -Prognose, ob ein Proband ein anomales Atemwegsepithel aufweist oder ob ein Proband, der ein anomales Atemwegsepithel aufweist, an Atemwegskrebs erkranken wird, unter Verwendung eines Kits, wobei das Kit Komponenten zum Bestimmen der Expressionsniveaus von mindestens drei Zielgenen eines TGF-$\beta$-Zellsignalwegs, mindestens drei Zielgenen eines PI3K-FOXO-Zellsignalwegs und mindestens drei Zielgenen eines Notch-Zellsignalwegs umfasst, wobei das Verfahren die Bereitstellung einer aus den Atemwegen des Probanden extrahierten Epithelzellprobe und das Unterziehen der extrahierten Epithelzellprobe dem Verfahren nach einem der Ansprüche 1 bis 7 umfasst.

14. Verfahren zur In-vitro- oder Ex-vivo-Diagnostik oder -Prognose, ob ein Proband ein anomales Atemwegsepithel aufweist oder ob ein Proband, der ein anomales Atemwegsepithel aufweist, an Atemwegskrebs erkranken wird, unter Verwendung eines Kits, wobei das Kit Komponenten zum Bestimmen der Expressionsniveaus von mindestens drei Zielgenen eines TGF-β-Zellsignalwegs, mindestens drei Zielgenen eines PI3K-FOXO-Zellsignalwegs und mindestens drei Zielgenen eines Notch-Zellsignalwegs umfasst, wobei das Verfahren Folgendes umfasst:

Bestimmen eines Atemweg-Anomaliefaktors, der angibt, ob der Proband ein anomales Atemwegsepithel aufweist, basierend auf einer Kombination von Aktivitäten zellulärer Signalwege in einer aus den Atemwegen des Probanden gewonnenen Epithelzellprobe,
wobei die zellulären Signalwege zwei oder mehr zelluläre Signalwege umfassen, die aus der Gruppe ausgewählt sind bestehend aus einem TGF-β-Signalweg, einem PI3K-F0X0-Signalweg und einem Notch-Signalweg,
wobei das Bestimmen des Signalweg-Anomaliefaktors weiter auf einer Referenzaktivität des jeweiligen zellulären Signalwegs basiert,
wobei die Referenzaktivität die Aktivität des jeweiligen zellulären Signalwegs im Atemwegsepithel gesunder Probanden widerspiegelt, und
wobei das Bestimmen des Atemweg-Anomaliefaktors Folgendes umfasst:
Bestimmen eines Signalweg-Anomaliefaktors für jeden der jeweiligen zellulären Signalwege auf Basis der Aktivität des jeweiligen zellulären Signalwegs in der Epithelzellprobe und Bestimmen des Atemweg-Anomaliefaktors auf Basis einer Kombination der bestimmten zellulären Signalweg-Anomaliefaktoren,
wobei eine quantitative Messung der Aktivität von Signaltransduktionswegen in Epithelzellen, die aus den oberen oder unteren Atemwegen erhalten wurden, auf dem Ableiten einer Aktivitätt eines Signaltransduktionswegs aus Messungen der mRNA-Spiegel von drei oder mehr validierten direkten Zielgenen des mit dem jeweiligen Signalweg assoziierten Transkriptionsfaktors basiert.

15. Verfahren zur *In-vitro-* oder Ex-vivo-Diagnostik oder -Prognose, ob ein Proband ein anomales Atemwegsepithel aufweist oder ob ein Proband, der ein anomales Atemwegsepithel aufweist, an Atemwegskrebs erkranken wird, nach Anspruch 13 oder 14, wobei

die drei oder mehr TGF-β-Zielgene aus der Gruppe ausgewählt sind, bestehend aus: ANGPTL4, CDC42EP3, CDKN1A, CTGF, GADD45A, GADD45B, HMGA2, ID1, IL11, JUNE, PDGFB, PTHLH, SERPINE1, SGK1, SKIL, SMAD4, SMAD5, SMAD6, SMAD7, SNAI2, VEGFA, bevorzugter aus der Gruppe bestehend aus: ANGPTL4, CDC42EP3, CDKN1A, CTGF, GADD45B, ID1, IL11, JUNB, SERPINE1, PDGFB, SKIL, SMAD7, SNAI2 und VEGFA, besonders bevorzugt aus der Gruppe bestehend aus: ANGPTL4, CDC42EP3, ID1, IL11, JUNB, SERPINE1, SKIL und SMAD7, oder wobei die drei oder mehr TGF-β-Zielgene aus der Gruppe ausgewählt sind, bestehend aus: CDC42EP3, GADD45B, HMGA2, ID1, JUNB, OVAL1, VEGFA, SGK1,
und/oder
die drei oder mehr PI3K-FOXO-Zielgene aus der Gruppe ausgewählt sind, bestehend aus: AGRP, BCL2L11, BCL6, BNIP3, BTG1, CAT, CAV1, CCND1, CCND2, CCNG2, CDKN1A, CDKN1B, ESRI, FASLG, FBXO32, GADD45A, INSR, MXI1, NOS3, PCK1, POMC, PPARGC1A, PRDX3, RBL2, SOD2, TNFSF10, bevorzugt aus der Gruppe bestehend aus: FBXO32, BCL2L11, SOD2, TNFSF10, BCL6, BTG1, CCNG2, CDKN1B, BNIP3, GADD45A, INSR und MXI1.
und/oder
die drei oder mehr Notch-Zielgene aus der Gruppe ausgewählt sind, bestehend aus: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9 und TNC, wobei bevorzugt zwei oder mehr Notch-Zielgene aus der Gruppe ausgewählt sind, bestehend aus: DTX1, HES1, HES4, HES5, HEY2, MYC, NRARP und PTCRA, und ein oder mehrere Notch-Zielgene aus der Gruppe ausgewählt sind, bestehend aus: CD28, CD44, DLGAP5, EPHB3, FABP7, GFAP, GIMAP5, HES7, HEY1, HEYL, KLF5, NFKB2, NOX1, PBX1, PIN1, PLXND1, SOX9 und TNC.

## Revendications

1. Procédé mis en œuvre par ordinateur pour déterminer si un sujet présente un épithélium anormal des voies respiratoires, réalisé par un dispositif de traitement numérique, dans lequel la détermination comprend les étapes consistant à :

déterminer un facteur d'anomalie des voies respiratoires indiquant si le sujet présente ou non un épithélium anormal des voies respiratoires, sur la base d'une combinaison d'activités de voies de signalisation cellulaire dans un échantillon de cellules épithéliales provenant des voies respiratoires du sujet,
dans lequel les voies de signalisation cellulaire comprennent deux voies de signalisation cellulaire ou plus sélectionnées dans le groupe consistant en une voie TGF-β, une voie PI3K-FOXO et une voie Notch,
dans lequel la détermination du facteur d'anomalie de voie de signalisation est basée en outre sur une activité de référence de la voie de signalisation cellulaire respective,
dans lequel l'activité de référence reflète une activité de la voie de signalisation cellulaire respective présente dans l'épithélium des voies respiratoires de sujets sains,
dans lequel la détermination du facteur d'anomalie des voies respiratoires comprend les étapes consistant à : déterminer un facteur d'anomalie de voie de signalisation pour chacune des voies de signalisation cellulaire respectives sur la base de l'activité de la voie de signalisation cellulaire respective dans l'échantillon de cellules épithéliales et déterminer le facteur d'anomalie des voies respiratoires sur la base d'une combinaison des facteurs d'anomalie de voie de signalisation cellulaire déterminés,
dans lequel une mesure quantitative d'une activité de voie de transduction de signal dans des cellules épithéliales obtenues à partir des voies respiratoires supérieures ou inférieures est basée sur une déduction d'une activité d'une voie de transduction de signal à partir de mesures de niveaux d'ARNm de trois gènes cibles directs validés ou plus du facteur de transcription associé à la voie de signalisation respective.

2. Procédé mis en œuvre par ordinateur pour déterminer un score de risque indiquant le risque qu'un sujet présentant un épithélium anormal des voies respiratoires développe un cancer des voies respiratoires, réalisé par un dispositif de traitement numérique, dans lequel la détermination comprend les étapes consistant à :

déterminer le score de risque sur la base d'une combinaison des activités des voies de signalisation cellulaire dans un échantillon de cellules épithéliales provenant des voies respiratoires du sujet,
dans lequel les voies de signalisation cellulaire comprennent deux voies de signalisation cellulaire ou plus sélectionnées dans le groupe consistant en une voie TGF-β, une voie PI3K-FOXO et une voie Notch,
dans lequel la détermination du score de risque est basée en outre sur une combinaison d'activités de référence des voies de signalisation cellulaire, et
dans lequel le score de risque est défini de telle sorte que le risque indiqué augmente avec une activité décroissante de la voie TGF-β et une ou plusieurs parmi une activité croissante de la voie PI3K, et/ou une activité décroissante de la voie Notch par rapport aux activités de référence des voies de signalisation cellulaire,
dans lequel la détermination du score de risque comprend les étapes consistant à : déterminer un facteur d'anomalie des voies respiratoires sur la base de la combinaison des activités des voies de signalisation cellulaire dans l'échantillon de cellules épithéliales et traduire le facteur d'anomalie des voies respiratoires en le score de risque,
dans lequel la détermination du facteur d'anomalie des voies respiratoires comprend les étapes consistant à : déterminer un facteur d'anomalie de voie de signalisation pour chacune des voies de signalisation cellulaire respectives sur la base de l'activité de la voie de signalisation cellulaire respective dans l'échantillon de cellules épithéliales et déterminer le facteur d'anomalie des voies respiratoires sur la base d'une combinaison des facteurs d'anomalie de voie de signalisation cellulaire déterminés,
dans lequel une mesure quantitative d'une activité de voie de transduction de signal dans des cellules épithéliales obtenues à partir des voies respiratoires supérieures ou inférieures est basée sur une déduction d'une activité d'une voie de transduction de signal à partir de mesures de niveaux d'ARNm de trois gènes cibles directs validés ou plus du facteur de transcription associé à la voie de signalisation respective.

3. Procédé selon l'une quelconque des revendications précédentes,
dans lequel le facteur d'anomalie des voies respiratoires et le score de risque sont déterminés respectivement sur la base d'une évaluation d'un modèle mathématique calibré reliant les activités des voies de signalisation cellulaire dans l'échantillon de cellules épithéliales au facteur d'anomalie des voies respiratoires et au score de risque, respectivement.

4. Procédé selon la revendication 3, dans lequel le modèle mathématique de voie calibré est un modèle probabiliste, de préférence un modèle de réseau bayésien, basé sur des probabilités conditionnelles reliant le niveau d'activité de l'élément TF associé à la voie de signalisation et les niveaux d'expression des trois gènes cibles ou plus, ou le modèle mathématique de voie calibré peut être basé sur une ou plusieurs combinaisons linéaires des niveaux d'expression des trois gènes cibles ou plus.

5. Procédé selon l'une quelconque des revendications précédentes,
dans lequel les activités des voies de signalisation cellulaire dans l'échantillon de cellules épithéliales sont déduites ou peuvent être déduites par l'intermédiaire d'un procédé comprenant les étapes consistant à :

recevoir des niveaux d'expression d'un et de préférence de trois gènes cibles ou plus de chacune des voies de signalisation cellulaire respectives,

déterminer un niveau d'activité d'un élément de facteur de transcription (TF) associé à une voie de signalisation cellulaire, l'élément TF associé à une voie de signalisation cellulaire commandant la transcription des un et de préférence trois gènes cibles ou plus, la détermination étant basée sur l'évaluation d'un modèle mathématique de voie calibré établissant un lien entre les niveaux d'expression du ou des gènes cibles et le niveau d'activité de la voie de signalisation cellulaire respective, et

- déduire l'activité de la voie de signalisation cellulaire respective sur la base du niveau d'activité déterminé de l'élément TF associé à la voie de signalisation cellulaire.

6. Procédé selon l'une quelconque des revendications précédentes,

dans lequel les voies de signalisation cellulaire comprennent la voie TGF-β et une ou plusieurs de la voie PI3K-FOXO et de la voie Notch,
de préférence la voie PI3K-FOXO et au moins la voie PI3K-FOXO.

7. Procédé selon l'une quelconque des revendications précédentes, dans lequel :
les trois gènes cibles de TGF-β ou plus sont sélectionnés dans le groupe consistant en :

ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, plus préférentiellement dans le groupe consistant en : ANGPTL4, CDC42EP3, CDKN1A, CTGF, GADD45B, ID1, IL11, JUNB, SERPINE1, PDGFB, SKIL, SMAD7, SNAI2 et VEGFA, le plus préférentiellement dans le groupe consistant en : ANGPTL4, CDC42EP3, ID1, IL11, JUNB, SERPINE1, SKIL et SMAD7, ou dans lequel les trois gènes cibles de TGF-β ou plus sont sélectionnés dans le groupe consistant en : CDC42EP3, GADD45B, HMGA2, ID1, JUNB, OVAL1, VEGFA, SGK1,
et/ou
les trois gènes cibles de PI3K-FOXO ou plus sont sélectionnés dans le groupe consistant en : AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2, TNFSF10, de préférence dans le groupe consistant en : FBXO32, BCL2L11, SOD2, TNFSF10, BCL6, BTG1, CCNG2, CDKN1B, BNIP3, GADD45A, INSR et MXI1,
et/ou
les trois gènes cibles de Notch ou plus sont sélectionnés dans le groupe consistant en : CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9 et TNC, de préférence dans lequel deux gènes cibles de Notch ou plus sont sélectionnés dans le groupe consistant en : DTX1, HES1, HES4, HES5, HEY2, MYC, NRARP et PTCRA, et un ou plusieurs gènes cibles de Notch sont sélectionnés dans le groupe consistant en : CD28, CD44, DLGAP5, EPHB3, FABP7, GFAP, GIMAP5, HES7, HEY1, HEYL, KLF5, NFKB2, NOX1, PBX1, PIN1, PLXND1, SOX9 et TNC.

8. Procédé selon l'une quelconque des revendications précédentes,
dans lequel le procédé comprend en outre les étapes consistant à :

fournir des preuves supplémentaires d'un nodule non diagnostique malin ou bénin, et/ou
prédire la pré-malignité de l'épithélium des voies respiratoires, et/ou
prédire si une personne présente ou non un risque élevé de développer un cancer des voies respiratoires, et/ou
prédire si une personne présente ou non un risque élevé de développer un cancer du poumon, et/ou
prédire si une personne présente ou non un risque élevé de développer un cancer du poumon épidermoïde, et/ou
prédire si une personne présente ou non un risque élevé de développer un adénocarcinome pulmonaire, et/ou
prédire si une personne peut bénéficier ou non d'une thérapie locale pour prévenir le développement d'un cancer, et/ou
prédire si un patient est atteint d'un cancer du poumon, et/ou
pronostiquer et/ou prédire, et/ou
prédire l'efficacité d'un médicament, par exemple de chimiothérapie et/ou de traitement hormonal, et/ou
surveiller l'efficacité d'un médicament, et/ou
décider d'une fréquence de surveillance ou, plus particulièrement, d'une fréquence de surveillance de réponse à la thérapie, et/ou

développer un médicament, et/ou

développer un essai, et/ou

prédire si une personne risque ou non de développer un cancer invasif des voies respiratoires, et/ou

prédire si une personne risque ou non une progression de la maladie, et/ou

prédire ou diagnostiquer si une personne présente ou non un risque réduit après traitement (par exemple, chimioprévention), et/ou

compléter des informations diagnostiques provenant d'autres modalités (par exemple, l'imagerie) et/ou autres pathologies et/ou tests génétiques, et/ou

déterminer un stade d'un cancer.

9. Appareil pour déterminer un facteur d'anomalie des voies respiratoires indiquant si un sujet présente ou non un épithélium anormal des voies respiratoires ou un score de risque qui indique un risque qu'un sujet présentant un épithélium anormal des voies respiratoires développe un cancer des voies respiratoires comprenant un processeur numérique configuré pour réaliser le procédé selon l'une quelconque des revendications 1 à 8.

10. Support de stockage non transitoire pour déterminer un facteur d'anomalie des voies respiratoires indiquant si un sujet présente ou non un épithélium anormal des voies respiratoires ou un score de risque qui indique un risque qu'un sujet présentant un épithélium anormal des voies respiratoires développe un cancer des voies respiratoires stockant des instructions de stockage qui peuvent être exécutées par un dispositif de traitement numérique pour réaliser le procédé selon l'une quelconque des revendications 1 à 8.

11. Programme informatique pour déterminer un facteur d'anomalie des voies respiratoires indiquant si un sujet présente ou non un épithélium anormal des voies respiratoires ou un score de risque qui indique un risque qu'un sujet présentant un épithélium anormal des voies respiratoires développe un cancer des voies respiratoires comprenant des moyens de code de programme pour amener un dispositif de traitement numérique à réaliser un procédé selon l'une quelconque des revendications 1 à 8, lorsque le programme informatique est exécuté sur le dispositif de traitement numérique.

12. Kit pour déterminer un facteur d'anomalie des voies respiratoires indiquant si un sujet présente ou non un épithélium anormal des voies respiratoires ou un score de risque qui indique un risque qu'un sujet présentant un épithélium anormal des voies respiratoires développe un cancer des voies respiratoires, le kit comprenant :

des composants permettant de déterminer les niveaux d'expression d'au moins trois gènes cibles d'une voie de signalisation cellulaire TGF-$\beta$, d'au moins trois gènes cibles d'une voie de signalisation cellulaire PI3K-FOXO, d'au moins trois gènes cibles d'une voie de signalisation cellulaire Notch, et

l'appareil selon la revendication 9, le support de stockage non transitoire selon la revendication 10 ou le programme informatique selon la revendication 11.

13. Procédé de diagnostic ou de pronostic *in vitro* ou *ex vivo* qu'un sujet présente ou non un épithélium anormal des voies respiratoires ou qu'un sujet présentant un épithélium anormal des voies respiratoires développe ou non un cancer des voies respiratoires à l'aide d'un kit, le kit comprenant des composants permettant de déterminer les niveaux d'expression d'au moins trois gènes cibles d'une voie de signalisation cellulaire TGF-$\beta$, d'au moins trois gènes cibles d'une voie de signalisation cellulaire PI3K-FOXO, d'au moins trois gènes cibles d'une voie de signalisation cellulaire Notch,

le procédé comprenant la fourniture d'un échantillon de cellules épithéliales extraites des voies respiratoires du sujet, et la soumission de l'échantillon de cellules épithéliales extraites au procédé selon l'une quelconque des revendications 1 à 7.

14. Procédé de diagnostic ou de pronostic in vitro ou ex vivo qu'un sujet présente ou non un épithélium anormal des voies respiratoires ou qu'un sujet présentant un épithélium anormal des voies respiratoires développe ou non un cancer des voies respiratoires à l'aide d'un kit, le kit comprenant des composants permettant de déterminer les niveaux d'expression d'au moins trois gènes cibles d'une voie de signalisation cellulaire TGF-$\beta$, d'au moins trois gènes cibles d'une voie de signalisation cellulaire PI3K-FOXO, d'au moins trois gènes cibles d'une voie de signalisation cellulaire Notch, le procédé comprenant les étapes consistant à :

déterminer un facteur d'anomalie des voies respiratoires indiquant si le sujet présente ou non un épithélium anormal des voies respiratoires, sur la base d'une combinaison d'activités de voies de signalisation cellulaire dans un échantillon de cellules épithéliales provenant des voies respiratoires du sujet,

dans lequel les voies de signalisation cellulaire comprennent deux voies de signalisation cellulaire ou plus sélectionnées dans le groupe consistant en une voie TGF-$\beta$, une voie PI3K-FOXO et une voie Notch,

dans lequel la détermination du facteur d'anomalie de voie de signalisation est basée en outre sur une activité de référence de la voie de signalisation cellulaire respective,

dans lequel l'activité de référence reflète une activité de la voie de signalisation cellulaire respective présente dans l'épithélium des voies respiratoires de sujets sains, et

dans lequel la détermination du facteur d'anomalie des voies respiratoires comprend les étapes consistant à :

déterminer un facteur d'anomalie de voie de signalisation pour chacune des voies de signalisation cellulaire respectives sur la base de l'activité de la voie de signalisation cellulaire respective dans l'échantillon de cellules épithéliales et déterminer le facteur d'anomalie des voies respiratoires sur la base d'une combinaison des facteurs d'anomalie de voie de signalisation cellulaire déterminés,

dans lequel une mesure quantitative d'une activité de voie de transduction de signal dans des cellules épithéliales obtenues à partir des voies respiratoires supérieures ou inférieures est basée sur une déduction d'une activité d'une voie de transduction de signal à partir de mesures de niveaux d'ARNm de trois gènes cibles directs validés ou plus du facteur de transcription associé à la voie de signalisation respective.

15. Procédé de diagnostic ou de pronostic *in vitro* ou *ex vivo* qu'un sujet présente ou non un épithélium anormal des voies respiratoires ou qu'un sujet présentant un épithélium anormal des voies respiratoires développe ou non un cancer des voies respiratoires selon la revendication 13 ou 14, dans lequel

les trois gènes cibles de TGF-$\beta$ ou plus sont sélectionnés dans le groupe consistant en : ANGPTL4, CDC42EP3, CDKN1A, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, plus préférentiellement dans le groupe consistant en : ANGPTL4, CDC42EP3, CDKN1A, CTGF, GADD45B, ID1, IL11, JUNB, SERPINE1, PDGFB, SKIL, SMAD7, SNAI2 et VEGFA, le plus préférentiellement dans le groupe consistant en : ANGPTL4, CDC42EP3, ID1, IL11, JUNB, SERPINE1, SKIL et SMAD7, ou dans lequel les trois gènes cibles de TGF-$\beta$ ou plus sont sélectionnés dans le groupe consistant en : CDC42EP3, GADD45B, HMGA2, ID1, JUNB, OVAL1, VEGFA, SGK1,

et/ou

les trois gènes cibles de PI3K-FOXO ou plus sont sélectionnés dans le groupe consistant en : AGRP, BCL2L11, BCL6, BNIP3, BTG1, CAT, CAV1, CCND1, CCND2, CCNG2, CDKN1A, CDKN1B, ESR1, FASLG, FBXO32, GADD45A, INSR, MXI1, NOS3, PCK1, POMC, PPARGC1A, PRDX3, RBL2, SOD2, TNFSF10, de préférence dans le groupe consistant en : FBXO32, BCL2L11, SOD2, TNFSF10, BCL6, BTG1, CCNG2, CDKN1B, BNIP3, GADD45A, INSR et MXI1,

et/ou

les trois gènes cibles de Notch ou plus sont sélectionnés dans le groupe consistant en : CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9 et TNC, de préférence dans lequel deux gènes cibles de Notch ou plus sont sélectionnés dans le groupe consistant en : DTX1, HES1, HES4, HES5, HEY2, MYC, NRARP et PTCRA, et un ou plusieurs gènes cibles de Notch sont sélectionnés dans le groupe consistant en : CD28, CD44, DLGAP5, EPHB3, FABP7, GFAP, GIMAP5, HES7, HEY1, HEYL, KLF5, NFKB2, NOX1, PBX1, PIN1, PLXND1, SOX9 et TNC.

Fig. 1A

Fig. 1B

Fig. 1C

Fig. 2A

EP 3 970 149 B1

Fig. 2B

41

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016062891 A1
- WO 2013011479 A
- WO 2014102668 A
- WO 2015101635 A
- WO 2016062891 A
- WO 2017029215 A
- WO 2014174003 A
- WO 2016062892 A
- WO 2016062893 A
- WO 2018096076 A
- EP 16200697
- EP 17194288
- EP 17194291
- EP 17194293
- EP 17209053
- EP 2018076232 W
- EP 2018076334 W
- EP 2018076488 W
- EP 2018076513 W
- EP 2018076614 W
- WO 2013011479 A2
- WO 2014102668 A2

**Non-patent literature cited in the description**

- *NLST Research Team. N Engl J Med;*, 2013, vol. 368, 1980-1991
- *Oncotarget*, 29 September 2017, vol. 8 (44), 78044-78056
- *Lung Cancer.*, November 2015, vol. 90 (2), 121-127
- *Respiration.*, 2011, vol. 81 (4), 265-84
- *Clin Cancer Res.*, 01 July 2018, vol. 24 (13), 2984-2992
- *Sci Transl Med.*, 07 April 2010, vol. 2 (26), 26-25
- **W VERHAEGH et al.** *Cancer research*, 2014, vol. 74 (11), 2936-45
- **A VAN DE STOLPE et al.** *Scientific Reports*, 2019, vol. 9, 1603
- **VAN OOIJEN.** *Am J Pathol*, 2018, vol. 188 (9), 1956-1972
- **TILLEY et al.** *J Respir Crit Care Med.*, 15 March 2009, vol. 179 (6), 457-66
- **HWANG et al.** *J Immunol.*, 15 July 2011, vol. 187 (2), 987-98
- **SAMANTA et al.** *Cancer Prev Res (Phila).*, March 2012, vol. 5 (3), 453-63
- **W VERHAEGH** ; **A VAN DE STOLPE.** *Oncotarget*, 2014, vol. 5 (14), 5196
- **MASSAGUE et al.** *Cell*, 2008, vol. 134 (2), 215-230
- **SAMANTA et al.** *Cancer Prev Res*, 2012, vol. 5 (3), 453-63
- **HWANG et al.** *J Immunol*, 2011, vol. 187 (2), 987-998
- **ZOU et al.** *Oncology Letters*, 2018, vol. 15, 3415-3421
- *Cancer Discov*, 2014, vol. 4 (11), 1252
- **CHEN et al.** *Journal of Cancer*, 2017, vol. 8 (7), 1292-1300
- *Nat Rev Clin Oncol*, 2017, vol. 14 (9), 549-561
- **TILLEY et al.** *Am J Respir Crit Care Med*, 2009, vol. 179 (6), 457-66
- **SHAYKHIEV R** ; **WANG R** ; **ZWICK RK** ; **HACKETT NR et al.** Airway basal cells of healthy smokers express an embryonic stem cell signature relevant to lung cancer. *Stem Cells*, September 2013, vol. 31 (9), 1992-2002